# EUROPEAN PATENT SPECIFICATION

(11) **EP 1 351 709 B1**
(45) Date of publication and mention of the grant of the patent: **15.09.2004**
(21) Application number: 01948294.2
(22) Date of filing: 08.06.2001
(51) Int. Cl.: A61K 47/48, A61P 35/00

(54) **PEGYLATION OF LINKERS IMPROVES ANTITUMOR ACTIVITY AND REDUCES TOXICITY OF IMMUNOCONJUGATES**
PEGYLATION VON LINKER VERBESSERT DIE ANTITUMOR-AKTIVITÄT UND VERRINGERT DIE TOXIZITÄT VON IMMUNKONJUGATEN
PEGYLATION DE LIEURS AMELIORANT L'ACTIVITE ANTITUMORALE ET REDUISANT LA TOXICITE D'IMMUNOCONJUGUES

(30) Priority: 09.06.2000 US 211331 P; 22.06.2000 US 213804 P
(43) Date of publication of application: 15.10.2003
(73) Proprietor: The Government of The United States of America, as represented by The Department of Health and Human Services, Bethesda, MD 20892 (US); Kreitman, Robert J., Potomac, MD 20854 (US)
(72) Inventor: PASTAN, Ira, H., Potomac, MD 20854 (US); TSUTSUMI, Yasuo, Rockville, MD 20852 (US); ONDA, Masanori, Rockville, MD 20852 (US); NAGATA, Satoshi, Rockville, MD 20852 (US); LEE, Byungkook, Potomac, MD 20854 (US)
(74) Representative: Thomson, Paul Anthony
(86) International application number: PCT/US2001/018503
(87) International publication number: WO 2001/095942

(56) References cited:
- Y. TSUTSUMI ET AL: "Site-specific chemical modification with polyethylene glycol of recombinant immunotoxin anti-Tac(Fv)-PE38 (LMB-2) improves antitumor activity and reduces animal toxicity and immunogenicity" PROCEEDINGS OF THE NATIONAL ACADEMY OF SCIENCES, vol. 97, no. 15, 18 July 2000 (2000-07-18), pages 8548-8553, XP002227760
- R. J. KREITMAN ET AL: "Complete regression of human B-cell lymphoma xenografts in mice treated with recombinant anti-CD22 immunotoxin RFB4(dsFv)-PE38 at doses tolerated by cynomolgus monkeys" INTERNATIONAL JOURNAL OF ACNCER , vol. 81, 1999, pages 148-155, XP002227761 cited in the application
- R. J. KREITMAN ET AL: "Responses in Refractory Hairy Cell Leukemia to a Recombinant Immunotoxin" BLOOD, vol. 94, no. 10, 15 November 1999 (1999-11-15), pages 3340-3348, XP002227762 cited in the application
- MILTON HARRIS J ET AL: "PEGYLATION A NOVEL PROCESS FOR MODIFYING PHARMACOKINETICS" CLINICAL PHARMACOKINETICS, LEA & FEBIGER, PHILADELPHIA, PA, US, vol. 40, no. 7, 2001, pages 539-551, XP001106431
- REITER Y ET AL: "IMPROVED BINDING AND ANTITUMOR ACTIVITY OF A RECOMBINANT ANTI-ERBB2IMMUNOTOXIN BY DISULFIDE STABILIZATION OF THE FV FRAGMENT" JOURNAL OF BIOLOGICAL CHEMISTRY, AMERICAN SOCIETY OF BIOLOGICAL CHEMISTS, BALTIMORE, MD, US, vol. 269, no. 28, 15 July 1994 (1994-07-15), pages 18327-18331, XP000941643 ISSN: 0021-9258 cited in the application
- CHIEN-TSUN KUAN ET AL: "PSEUDOMONAS EXTOXIN A MUTANTS REPLACEMENT OF SURFACE EXPOSED RESIDUES IN DOMAIN IN WITH CYSTEINERESIDUES THAT CAN BE MODIFIED WITH POLYETHYLENE GLYCOL IN A SITE-SPECIFIC MANNER" JOURNAL OF BIOLOGICAL CHEMISTRY, AMERICAN SOCIETY OF BIOLOGICAL CHEMISTS, BALTIMORE, MD, US, vol. 269, no. 10, 11 March 1994 (1994-03-11), pages 7610-7616, XP000563744 ISSN: 0021-9258 cited in the application
- DATABASE MEDLINE [Online] October 1996 (1996-10) TSUTSUMI Y ET AL: "Molecular design of hybrid tumour necrosis factor-alpha. II: The molecular size of polyethylene glycol-modified tumour necrosis factor-alpha affects its anti-tumour potency." Database accession no. NLM8855980 XP002227763 cited in the application & BRITISH JOURNAL OF CANCER. SCOTLAND OCT 1996, vol. 74, no. 7, October 1996 (1996-10), pages 1090-1095, ISSN: 0007-0920

## Description

### CROSS-REFERENCES TO RELATED APPLICATIONS

This application claims the benefit of U.S. Provisional Patent Applications 60/211,331, filed June 9, 2000, and 60/213,804, filed June 22, 2000. The contents of both of these applications are incorporated herein by reference for all purposes.

### STATEMENT AS TO RIGHTS TO INVENTIONS MADE UNDER FEDERALLY SPONSORED RESEARCH AND DEVELOPMENT

Not applicable.

### BACKGROUND OF THE INVENTION

Recombinant immunoconjugates are chimeric molecules in which a molecule with an intended function, termed the "effector molecule," is coupled to a targeting molecule which targets the conjugate, typically to a cell expressing a particular receptor or antigen recognized by the targeting molecule. Immunotoxins are a form of immunoconjugate in which a toxin, usually truncated, serves as the effector molecule, and is fused to an Fv portion of an antibody or to a ligand that serves as the targeting moiety. Many different recombinant immunotoxins in which the Fv portion of an antibody to a tumor-related antigen is fused to a 38 kDa mutant form of *Pseudomonas* exotoxin A (PE) which has a deletion of its cell binding domain have been produced (Pastan (1997) *Biochim. Biophys. Acta. 24:*1333; Kreitman *et al*. (1994) *Blood 83*:426-434; Kreitman *et al*, (1999) *Int. J. Cancer 81*:148-155; Brinkmann *et al*. (1991) *Proc. Natl. Acad. Sci. USA 88*:8616-8620; Reiter *et al*. (1994) *Cancer Res*. *54*:2714-2718; Reiter *et al*. (1994) *J. Biol. Chem. 269*:18327-18331). Five of these recombinant immunotoxins, (anti-Tac(Fv)-PE38, B3(Fv)-PE38, B3(dsFv)-PE38, RFB4(dsFv)-PE38, and e23(dsFv)-PE38) have recently been evaluated in Phase I trials in patients with cancer (Kreitman *et al*. (1999) *Blood 94*:3340-3348; Pai-Scherf *et al*. (1999) *Clin. Cancer Res. 5*:2311-2315). All of these immunotoxins have produced complete regressions of human cancer xenografts in nude mice and are relatively well tolerated by mice and monkeys.

Anti-Tac(Fv)-PE38 (also known as "LMB-2"), which comprises the Fv fragment of the anti-human Tac monoclonal antibody coupled to the IL-2 receptor α subunit (also referred to as Tac, p55, or CD25) has produced major clinical responses in hematologic malignancies (Kreitman *et al*. (1999) *Blood 94*:3340-3348). LMB-2 was administered to 35 patients with CD25+ hematologic malignancies who had failed standard and salvage therapies. One patient with hairy cell leukemia (HCL) had a complete remission, ongoing at 20 months, and seven partial responses were observed in HCL, cutaneous T-cell lymphoma, chronic lymphocytic leukemia, Hodgkin's disease and adult T-cell leukemia. See, Kreitman et al., (2000) J. Clin Oncol 18(8):1622-36.

However, LMB-2 exhibited side-effects which limited the amount of immunotoxin that could be given to patients. These toxic side-effects are at least in part due to the nonspecific binding of LMB-2 to normal tissues, because one dose-limiting toxicity was damage to liver cells which do not express IL-2 receptors (Kreitman and Pastan (1995) *Semin. Cancer Biol. 5*:297-306.). Side-effects resulting from specific targeting of CD25+ normal cells may also have occurred. In addition, human anti-PE antibodies and occasionally anti-mouse Fv antibodies were formed in some patients treated with LMB-2. This immunogenicity of recombinant immunotoxins reduces their therapeutic usefulness. If side-effects and immunogenicity can be prevented, one should be able to give more immunotoxin and obtain improved responses in human malignancies. A new strategy designed to decrease the side effects of LMB-2 in which mutations are introduced into the framework region of the Fv in order to lower its isoelectric point was recently described (Onda *et al*. (1999) *J. Immunol. 163*:6072-6077). These mutant immunotoxins are less toxic to mice, allowing higher doses to be given with a substantial increase in antitumor activity. Liver damage was still, however, the dose limiting toxicity and this approach is not designed to decrease immunogenicity.

These recombinant immunotoxins (Mw: 63,000) have a lower molecular weight than conventional antibody-toxin conjugates (Mw: ∼190,000). While lower molecular weight molecules have increased distribution into tumors (as well as various normal tissues such as kidney and liver), they are also removed from the circulation faster than are larger molecules. Preclinical studies have shown that antitumor activity is enhanced if recombinant immunotoxins survive longer in the circulation (Pai *et al*. (1991) *Cancer Res. 51*:2808-2812). Thus, an increase in blood-residency time of lower weight immunotoxins should lead to an increase in their antitumor activities. The net result would be an augmentation of their therapeutic potency.

One way to increase the blood-residency of proteins is to modify them with polyethylene glycol (PEG). Chemical modification of proteins with PEG (PEGylation) increases their molecular size and steric hindrance, both of which are dependent on the PEG attached to the protein. This results in an improvement of plasma half-lives and in proteolytic-stability, and a decrease in immunogenicity and hepatic uptake (Chaffee *et al*. (1992) *J. Clin. Invest. 89*:1643-1651; Pyatak *et al*. (1980) *Res. Commun. Chem. Pathol Pharmacol. 29*:113-127). PEGylation of interleukin-2 has been reported to increase its antitumor potency *in vivo* (Katre *et al*. (1987) *Proc. Natl. Acad. Sci. USA. 84*:1487-1491) and PEGylation of an F(ab')2 derived from the monoclonal antibody A7 has improved its tumor localization (Kitamura *et al*. (1990) *Biochem. Biophys. Res. Commun. 28*:1387-1394).

We previously reported that a PEGylated chimeric toxin composed of transforming growth factor-α and PE showed an improvement in its blood-residency time and a decrease in its immunogenicity resulting in enhanced *in vivo* antitumor potency and reduced *in vivo* toxicity (Wang *et al*. (1993) *Cancer Res. 53*:4588-4594). However, we also found that PEGylation was accompanied by an undesirable and significant loss of cytotoxic activity to the targeted cells. Unlike PEGylation of enzymes, which act on small substrates, PEGylation of recombinant immunotoxins may cause a decrease in their activity due to loss of antigen-binding, of translocation to the cytosol, or of ADP-ribosylation activity, because these steps are based on macromolecular interactions which are easily sterically hindered by the attached PEG. In most cases, PEGylation of proteins is non-specific and targeted at all the lysine residues in the protein, some of which may be in or near the active-site. To overcome this drawback, site-specific PEGylation of mutant PE molecules that were engineered to contain one or two cysteine residues on the surface ofPE was attempted (Benhar *et al*. (1994) *J. Biol. Chem. 269*:13398-133404; Kuan et al. (1994) *J. Biol. Chem. 269*:7610-7616). Free thiol chemistry was used for the attachment of PEG to these residues. This approach proved unsuccessful, resulting in a low yield of PEGylated immunotoxin and a significant loss in activity.

There is therefore a need in the art for immunotoxins and other immunoconjugates having decreased toxicity, while preserving antitumor activity.

### SUMMARY OF THE INVENTION

The present invention is directed to the site-directed PEGylation of immunoconjugates, including immunotoxins. In particular, the present invention provides a new approach for modifying with polyethylene glycol (PEG) a connector molecule that attaches the toxin moiety to the targeting moiety of an immunotoxin. In some aspects of the invention, a mutant of the immunotoxin of interest is prepared in which one or more cysteines are introduced in a peptide connector that attaches the antibody to the toxin, and the mutant is then modified with PEG-maleimide. The PEGylated immunotoxin has comparable *in vitro* specific cytotoxicity against tumor cells, but other properties including stability, plasma half-life, antitumor activity, immunogenicity and non-specific toxicity are greatly improved.

In one aspect, the present invention is directed to compositions comprising an antibody or fragment thereof retaining antigen recognition capability, linked to a toxin through a connector molecule, wherein the connector molecule comprises one or more polyethylene glycol molecules. In one embodiment, the connector molecule is a peptide. In another embodiment, the toxin is the *Pseudomonas* exotoxin (PE). In a preferred embodiment, the toxin is a portion of the *Pseudomonas* exotoxin which retains ADP-ribosylation activity, preferably a 38 kDa fragment (PE38). In yet another embodiment, the antibody portion of the immunotoxin is a single-chain Fv fragment of the anti-human Tac monoclonal antibody. In preferred embodiments, the immunotoxin is anti-Tac(Fv)-PE38 (*i.e*., LMB-2)

The present invention further provides a method of increasing anti-tumor activity of an immunotoxin having a targeting moiety and a toxin moiety connected by a connector molecule, said method comprising covalently bonding a polyethylene glycol molecule to said connector molecule. The toxin moiety can be selected from the group consisting of *Pseudomonas* exotoxin (PE) or a fragment or mutant thereof which retains cytotoxic activity, *Diphtheria* toxin or a fragment or mutant thereof which retains cytotoxic activity, ricin, saponin, gelonin, ribosome inactivating protein, abrin, and botulinum A-F. In a preferred embodiment, the toxin is a *Pseudomonas* exotoxin (PE). In a more preferred embodiment, the toxin is a 38 kDa fragment from *Pseudomonas* exotoxin (PE38). In yet another embodiment, the antibody portion of the immunotoxin is a single-chain Fv fragment of the anti-human Tac monoclonal antibody. In especially preferred embodiments, the immunotoxin is anti-Tac(Fv)-PE38 (*i.e.*, LMB-2).

In particular, the invention provides compositions comprising a targeting molecule linked to an effector molecule through a connector molecule, which connector molecule comprises one or more polyethylene glycol (PEG) molecules. The targeting molecule can be selected from a ligand, an antibody, and a fragment of an antibody which fragment retains antigen recognition capability. The antibody or fragment thereof which retains antigen recognition capability can specifically recognize the IL-2 receptor α subunit. In some embodiments, the effector molecule is a toxin, and in preferred embodiments is a *Pseudomonas* exotoxin or a portion thereof which retains ADP-ribosylation activity ("PE"). In a particularly preferred embodiment, the PE is PE38.

In preferred embodiments, the connector molecule is a peptide. While the peptide may be between 4-100 amino acids and is preferably shorter than about 50 amino acids, in preferred embodiments, the connector molecule can be ASGGPE (SEQ ID NO:1) mutated by site specific mutagenesis to contain a cysteine, as in ASGCGPE (SEQ ID NO:2), and ASGCCGPE (SEQ ID NO:3), or can be, for example, ASCGSGCPE (SEQ ID NO:4), KASGKKYGCKKGPE (SEQ ID NO:5), ASCGTTGCPE (SEQ ID NO:8), or KGGGCAGGPE (SEQ ID NO:6).

The PEG molecule is substituted for a reactive group on an amino acid residue of the connector molecule. The PEG molecule can have a molecular weight of between 1 and 100 kD, more preferably has a molecular weight between about 3 kD and 50 kD, even more preferably has a molecular weight of between about 5 kD and about 20 kD. In a particularly preferred embodiment, the targeting molecule is an anti-IL-2 receptor α subunit antibody known as anti-Tac, wherein said effector molecule is PE38, and said connector molecule is ASGCGPE (SEQ ID NO:2).

In one group of embodiments, the invention provides compositions comprising the compositions noted above in a pharmaceutically acceptable carrier.

In another group of embodiments, the invention provides methods of increasing anti-tumor activity of an immunotoxin having a targeting moiety and a toxin moiety connected by a connector molecule, said method comprising covalently bonding a polyethylene glycol molecule to said connector molecule. Two or more residues of said connector molecule may be conjugated to PEG molecules (that is, each residue is separately conjugated to a PEG molecule which is separate from the PEG molecule to which the other residue is conjugated). The targeting molecule can be selected from a ligand, an antibody, and a fragment of an antibody which fragment retains antigen recognition capability. In some preferred embodiments, the antibody or fragment thereof specifically recognizes the IL-2 receptor α subunit. The effector molecule can be a toxin; in particular, the effector can be a *Pseudomonas* exotoxin (PE) or a portion thereof which retains ADP-ribosylation activity. In a preferred embodiment, the PE is PE38.

The connector molecule can be a peptide. In particular, the connector molecule can be any of the following peptides, although the position of the cysteine can be changed if desired: ASGCGPE (SEQ ID NO:2), ASGCCGPE (SEQ ID NO:3), ASCGSGCPE (SEQ ID NO:4), KASGKKYGCKKGPE (SEQ ID NO:5), ASCGTTGCPE (SEQ ID NO:8), and KGGGCAGGPE (SEQ ID NO:6).

The immunoconjugates of the invention are PEGylated by substituting for a PEG molecule for a reactive group on one or more amino acid residues of the connector molecule. The PEG molecule can have a molecular weight (Mw) of between 1 and 100 kD, typically has a Mw of between about 3 kD and about 50 kD, and preferably has a molecular weight of between about 5 kD and about 20 kD.

The effector molecule can be a cytotoxin, a label, a radionuclide, a detectable label, a drug, a liposome, a nucleic acid, a recombinant virus, a glycoprotein, a ligand, or and an antibody. The cytotoxin can be *Pseudomonas* exotoxin A (PE) or a fragment or mutant thereof which retains cytotoxic activity, *Diphtheria* toxin or a fragment or mutant thereof which retains cytotoxic activity, ricin, saponin, gelonin, ribosome inactivating protein, abrin, or a botulinum toxin A-F.

In a preferred embodiment, the targeting moiety is an anti-CD35 antibody known as anti-Tac, the effector molecule is PE38, and the connector molecule is ASGCGPE (SEQ ID NO:2).

### BRIEF DESCRIPTION OF THE DRAWINGS

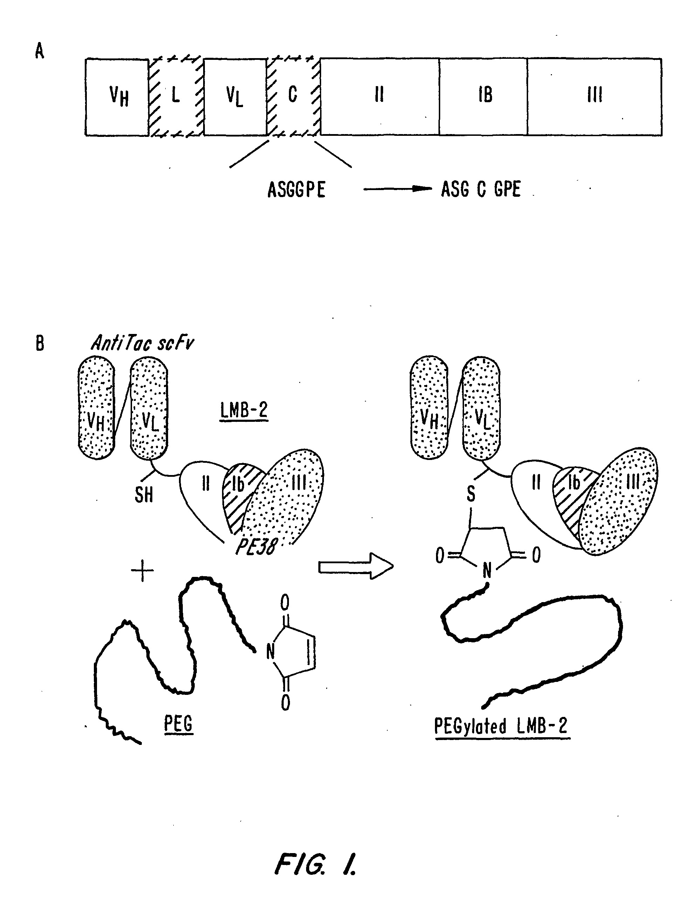

**Figure 1** (A) Schematic representation of mutant LMB-2 (anti-Tac(Fv)-PE38) with one cysteine in the connector. The positions of amino acids that span PE sequences are numbered. The amino acid sequence of the connector (C) of mutant LMB-2 (cys1-LMB-2) is changed from ASGGPE (SEQ ID NO:1) to ASGCGPE (SEQ ID NO:2). V_{H} refers to the heavy chain fragment of the anti-Tac antibody; L represents to the peptide linker (GGGGS)₃ (SEQ ID NO:7); V_{L} refers to the light chain fragment; C represents the connector; II is PE domain II; Ib is PE domain Ib; and III is PE domain III.
   (B) The site-specific PEGylation to a cysteine residue in the connector of the LMB-2 mutant. cys1-LMB-2 is site-specifically conjugated with PEG via the formation of a thioether bond between a free thiol group in the connector of the LMB-2 mutant and the maleimide at one end of the PEG chain.

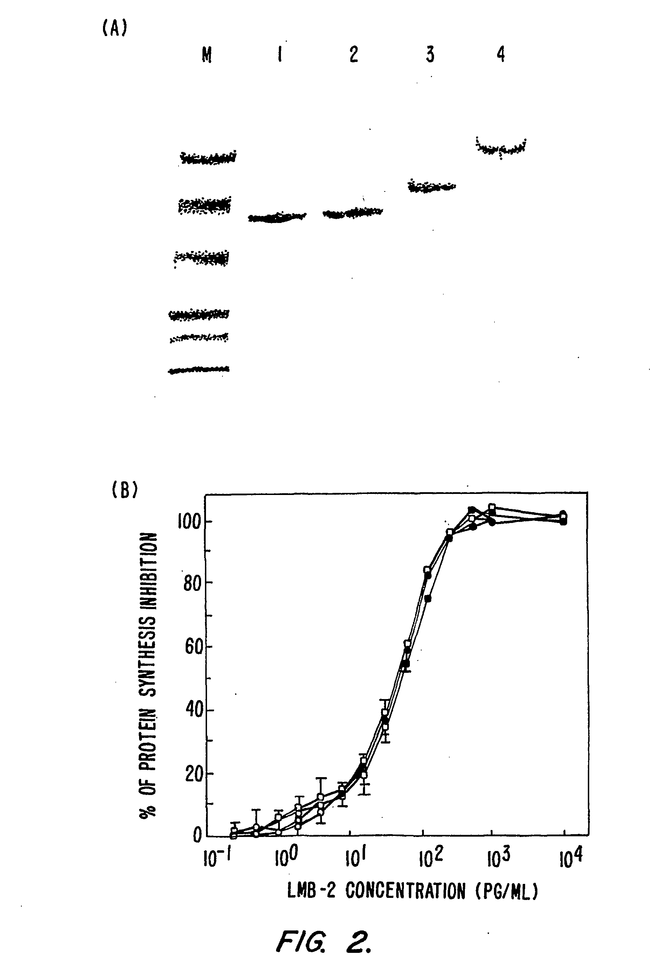

Figure 2 Properties of the LMB-2 molecules.
   (A) SDS-PAGE analysis of PEGylated LMB-2s. SDS-PAGE was performed under non-reducing conditions. Lane M shows the molecular weight standards, which from top to bottom, are Mw 111,000, 73,000, 47,500, 33,900, 28,800, and 20,500; Lane 1, 2, 3, and 4 correspond to LMB-2; cys1-LMB-2; PEG5K-LMB-2; and PEG20K-LMB-2, respectively.
   (B) *In vitro* specific cytotoxicity of various forms of LMB-2 on ATac4 cells. Various LMB-2s were diluted with 0.2% BSA in DPBS. ATac-4 cells were seeded at 2.0 x 10⁴ cells/well in 96-well plates, 24 hr prior to the addition of LMB-2 (o), cys1-LMB-2 (●), PEG5K-LMB-2 (□), PEG20K-LMB-2 (■), and the incubation with them at 37°C for 24 hr. The cells were then assayed by measuring inhibition of incorporation of ³H-leucine.

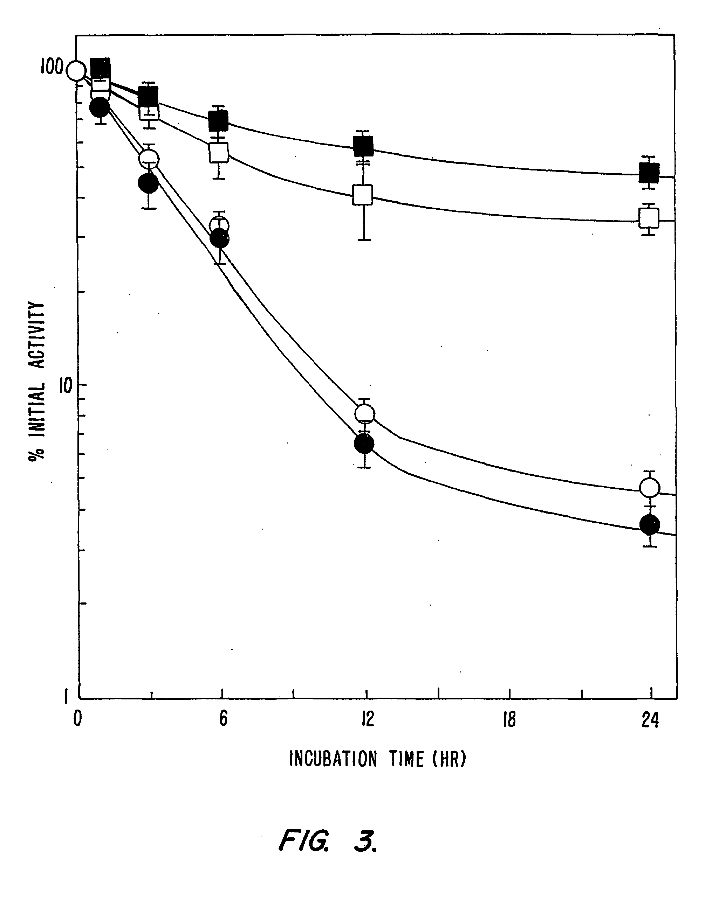

**Figure 3** Stability of PEGylated LMB-2s in mouse serum. The stability of LMB-2 (○), cys1-LMB-2 (●), PEG5K-LMB-2 (□), PEG20K-LMB-2 (■) was determined by incubation at 10 µg/ml at 37°C in mouse serum from female BALB/c mice (50 µl of 40 µg/ml immunotoxin + 150 µl mouse serum). The amount of active immunotoxin remaining after different times of incubation was determined by a cytotoxicity assay.

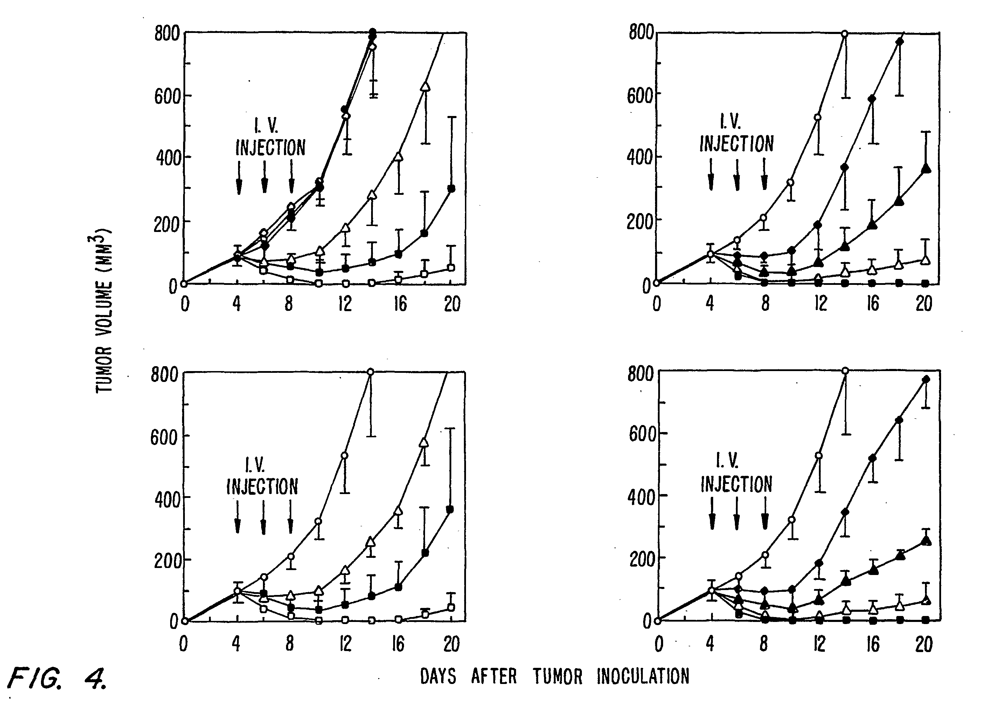

**Figure 4** Antitumor effects of PEGylated LMB-2s on ATac-4 solid tumors. ATac-4 cells (2 x 10₆) were inoculated subcutaneously on day 0 into nude mice. Starting on day 4, mice were treated with intravenous injections of immunotoxins on days 4, 6, 8. Groups of five mice were treated for each dose QOD x 3 at each dose level. (A) LMB-2, (B) cys1-LMB-2, (C) PEG5K-LMB-2 (D) PEG20K-LMB-2. The following symbols were used: ○, diluent; □, 0.100 mg/kg, ■, 0.050 mg/kg; Δ, 0.025; ▲, 0.013 mg/kg; ◆, 0.006 mg/kg, ●, PEG5K 0.5 mg/kg: ◇, PEG20K 0.5 mg/kg.

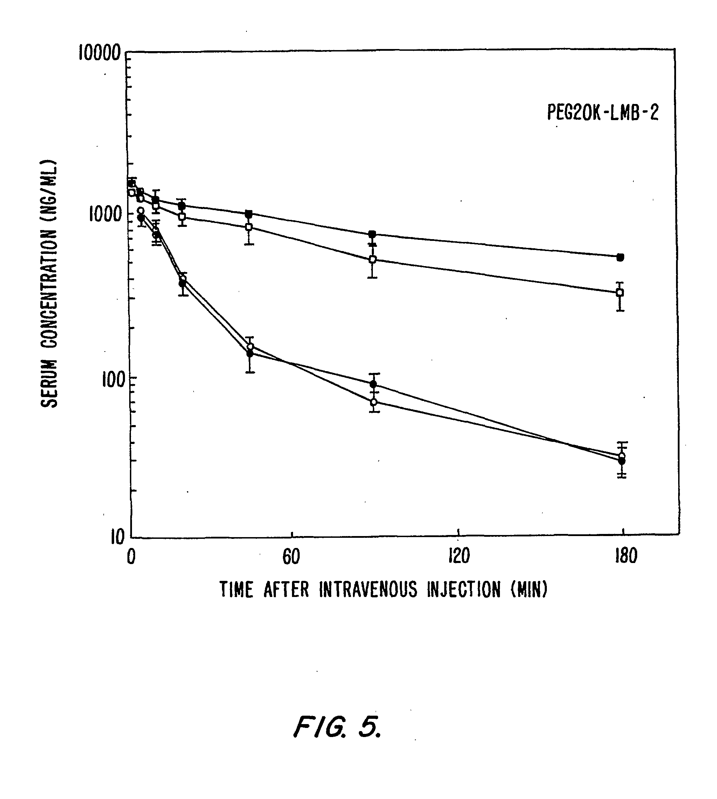

**Figure 5** Pharmacokinetics of PEGylated LMB-2s in mice. Normal female BALB/c mice were injected intravenously with 2 µg of LMB-2 (o), cys1-LMB-2 (●), PEG5K-LMB-2 (□), PEG20K-LMB-2 (■). Blood samples were drawn at different times. The level of immunotoxin was measured by bioassay in which diluted serum samples were incubated with Atac-4 cells, and the ability of serum samples to inhibit protein synthesis was measured. A standard curve was made for each immunotoxin. Groups of 4 mice were used. Values are mean ± SD.

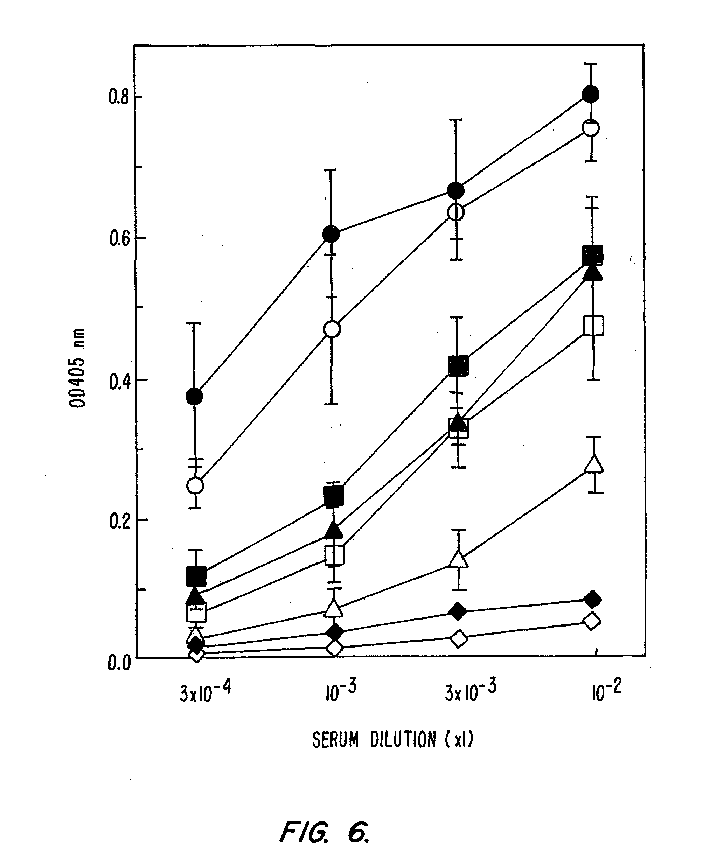

**Figure 6** The IgG responses in mice to PEGylated LMB-2s. Mice in groups of five were injected i.p. with LMB-2 (○), cys1-LMB-2 (●), PEG5K-LMB-2 (□), and PEG20K-LMB-2 (■) at a dose of 4 µg/mouse or 40 µg/mouse in PBS containing 0.2% mouse serum albumin (MSA). Two weeks after the first immunization, mice were once again immunized with the appropriate antigen. The specific IgG levels were determined by ELISA on day 21.

### DESCRIPTION OF THE SPECIFIC EMBODIMENTS

### I. INTRODUCTION

Surprisingly, it has been discovered that PEGylation of the linker or connector portion of an immunotoxin increases the anti-tumor activity of the immunotoxin, while decreasing its toxicity and immunogenicity. The invention therefore provides a significant advance in the art by providing a means to increase the therapeutic window of immunotoxins. This result could not have been predicted since it could not be known whether PEGylation of the linker would interfere with the folding or activity of the immunotoxin and, in particular, with the function of the targeting or of the toxin moieties.

Even more surprisingly, it has been discovered that both small and large molecular weight (Mw) polyethylene glycols can be conjugated to the linker portion of an immunotoxin without adverse effect on activity. The teaching in the art up to the present has been that the activity of PEGylated proteins decreases with increasing molecular weight of the attached PEG because the steric hindrance due to the attached PEG increases with the increasing length of the PEG chain (Tsutsumi *et al*. (1996) *Br. J. Cancer 74*:1090-1095). Our studies demonstrated, however, that an immunotoxin PEGylated with a 20 kD form of PEG had almost the same activity as a like immunotoxin PEGylated with a 5 kD form. PEGylation of the linker thus permits the immunotoxin to be PEGylated with whatever Mw of PEG provides the construct with the degree of stability and serum circulation time desired by the particular practitioner, without the loss of activity previously thought to be associated with higher Mw forms of PEG.

Based on the results obtained with this immunotoxin, it is expected that targeting molecules linked to effector molecules other than toxins, such as drugs and metal chelates, will likewise display improved therapeutic effects less

In preferred embodiments, the linker to which the PEG is conjugated is a peptide, preferably of from about 3 to about 50 amino acids in length, with about 6 to about 20 being more preferred. A number of such linker peptides are known in the art and are suitable for use in the invention. Conveniently, the linker can be conjugated to PEG by incorporating a cysteine into the linker at a desired position, allowing a PEG derivative such as PEG maleimide to be conjugated by thiol chemistry. Reactive sites of other amino acid residues can, however, be utilized to conjugate appropriate PEG derivatives to the linker, as explained further below. In preferred embodiments, PEG is attached to a single amino acid residue in the linker; however, two or more residues of the linker can be PEGylated if desired. Such constructs should be tested, for example, by the assays set forth in the Examples, to ensure that such multiple PEGylation does not adversely affect the activity profile of the immunotoxin (that is, its serum circulation time, ability to bind to and kill target cells, and the like).

Accordingly, the present invention is directed to compositions in which a linker connecting the effector molecule and the targeting moieties of an immunoconjugate is PEGylated. In particular, the present invention provides compositions comprising immunotoxins, wherein the connector linking the targeting moiety to the toxin is PEGylated in a site-specific manner. The present invention is further directed to methods for increasing the antitumor activity of an immunotoxin, comprising attaching one or more polyethylene glycol molecules to the connector attaching the toxin to the targeting moiety.

In particular, the present invention provides a method for introducing a mutation in the connector region attaching the antibody or antibody fragment to the toxin of interest, typically to introduce a cysteine residue. The cysteine residue or other introduced residue can then be used for site-specific PEGylation of the connector region. In the case of the introduction of a cysteine residue, PEGylation can conveniently be accomplished using thiol chemistry. Chemistries for accomplishing PEGylation are discussed in more detail within.

In one set of embodiments, the toxin used in immunotoxins of the invention is *Pseudomonas* exotoxin A (PE). Typically, PE molecules used in such immunotoxins have been modified to reduce or eliminate non-specific binding and toxicity. In a preferred embodiment, the toxin is a 38 kDa cytotoxic truncated fragment of *Pseudomonas* exotoxin known as PE38. In another set of embodiments, the antibody portion of the immunotoxin is a single-chain Fv fragment of an anti-human Tac monoclonal antibody to the IL-2 receptor α subunit (also known as Tac). In especially preferred embodiments, the immunotoxin is the anti-Tac(Fv)-PE38 also known as LMB-2.

Although these are particularly preferred embodiments, numerous antigens expressed on cancer cells are known in the art, as are antibodies which specifically bind to those cancer antigens. Further, immunoconjugates and immunotoxins employing these antibodies are also known in the art. The present invention specifically contemplates that the stability and serum circulation time of these immunoconjugates and immunotoxins can be improved by PEGylation of their linker moieties.

### II. DEFINITIONS

Units, prefixes, and symbols are denoted in their Système International de Unites (SI) accepted form. Numeric ranges are inclusive of the numbers defining the range. Unless otherwise indicated, nucleic acids are written left to right in 5' to 3' orientation; amino acid sequences are written left to right in amino to carboxy orientation. The headings provided herein are not limitations of the various aspects or embodiments of the invention, which can be had by reference to the specification as a whole. Accordingly, the terms defined immediately below are more fully defined by reference to the specification in its entirety.

In the context of the present invention, the term "antibody" or "antibody fragment(s)" refers to polyclonal and monoclonal antibodies, an entire immunoglobulin or antibody or any functional fragment of an immunoglobulin molecule which binds to the target antigen and is defined further below. Examples of such functional entities include complete antibody molecules, antibody fragments, such as Fv, single chain Fv, complementarity determining regions (CDRs), V_{L} (light chain variable region), V_{H} (heavy chain variable region), Fab, F(ab)₂' and any combination of those or any other functional portion of an immunoglobulin peptide capable of binding to a target antigen (*see, e.g*., Pierce Catalog and Handbook, 1994-1995 (Pierce Chemical Co., Rockford, IL; Kuby, *Immunology*, 3^{rd} Ed., W.H. Freeman & Co., New York (1997)).

An antibody immunologically reactive with a particular antigen can be generated by recombinant methods such as selection of libraries of recombinant antibodies in phage or similar vectors, *see, e.g.,* Huse, *et al., Science* **246**:1275-1281 (1989); Ward, *et al., Nature* **341**:544-546 (1989); and *Vaughan, et al., Nature Biotech*. **14**:309-314 (1996), or by immunizing an animal with the antigen or with DNA encoding the antigen.

Typically, an immunoglobulin has a heavy and light chain. Each heavy and light chain contains a constant region and a variable region, (the regions are also known as "domains"). Light and heavy chain variable regions contain a "framework" region interrupted by three hypervariable regions, also called "complementarity-determining regions" or "CDRs". The extent of the framework region and CDRs have been defined (*see*, Kabat, E., *et al*., SEQUENCES OF PROTEINS OF IMMUNOLOGICAL INTEREST, U.S. Department of Health and Human Services, (1987). The sequences of the framework regions of different light or heavy chains are relatively conserved within a species. The framework region of an antibody, that is the combined framework regions of the constituent light and heavy chains, serves to position and align the CDRs in three dimensional space.

Antibodies exist, *e.g*., as intact immunoglobulins or as a number of well characterized fragments produced by digestion with various peptidases. Thus, for example, pepsin digests an antibody below the disulfide linkages in the hinge region to produce F(ab)'₂, a dimer of Fab which itself is a light chain joined to V_{H}-C_{H1} by a disulfide bond. The F(ab)'₂ may be reduced under mild conditions to break the disulfide linkage in the hinge region, thereby converting the F(ab)'₂ dimer into an Fab' monomer. The Fab' monomer is essentially an Fab with part of the hinge region (see, *Fundamental Immunology*, 3rd Ed., W.E. Paul, ed., Raven Press, N.Y. (1993)). While various antibody fragments are defined in terms of the digestion of an intact antibody, one of skill will appreciate that such fragments may be synthesized *de novo* either chemically or by utilizing recombinant DNA methodology. Thus, the term antibody, as used herein, also includes antibody fragments either produced by the modification of whole antibodies or those synthesized *de novo* using recombinant DNA methodologies. Furthermore, in the context of the present invention, the term "antibody" also includes genetically engineered forms such as chimeric antibodies (e.g., humanized murine antibodies), heteroconjugate antibodies (*e.g*., bispecific antibodies) and recombinant single chain Fv fragments (scFv), disulfide stabilized (dsFv) Fv fragments *(see,* U.S. Patent Application No. 08/077,252), or pFv fragments (see, U.S. Provisional Patent Application Nos. 60/042,350 and 60/048,848).

The term "single chain antibody" refers to an antibody wherein the genetic information encoding the functional fragments of the antibody are located in a single contiguous length of DNA. For a thorough description of single chain antibodies, *see, e.g.,* Bire *et al*., (1988) *Science 242*:423; and *Huston et al*., (1988) *Proc. Natl Acad. Sci. USA 85*:5879.

"Tac" refers to the IL-2 receptor α subunit. Antibodies that specifically bind to this subunit are referred to as "anti-Tac" antibodies.

In the context of the present invention, "chimeric proteins" refers to proteins in which two or more molecules that exist separately in their native state are joined together to form a single molecule having the desired functionality of all of its constituent molecules.

The term "immunoconjugate" includes reference to a covalent linkage of an effector molecule to a targeting molecule, either directly or through a connector or linker molecule. In the context of the present invention, the effector molecule and the targeting molecule are connected through a linker molecule. In many of the preferred embodiments of the invention, the effector molecule is a toxin; immunoconjugates incorporating a toxin molecule are more specifically designated by the term "immunotoxin."

The term "moiety" is used to refer to a portion of a molecule, which portion has an intended functionality. Thus, in an immunoconjugate, the targeting portion may be referred to as the targeting moiety, and in an immunotoxin, the incorporated toxin molecule may be referred to as a "toxin moiety."

A "therapeutic moiety" is the portion of an immunoconjugate intended to act as a therapeutic agent.

The term "therapeutic agent" includes any number of compounds currently known or later developed to act as anti-neoplastics, anti-inflammatories, cytokines, antiinfectives, enzyme activators or inhibitors, allosteric modifiers, antibiotics or other agents administered to induce a desired therapeutic effect in a patient. The therapeutic agent may also be a toxin, where the therapeutic effect intended is, for example, the killing of a cancer cell. It may further be, for example, a radioisotope, often a metal chelate, which is conjugated to the linker by chemistry standard in the art.

A "detectable label" means, with respect to an immunoconjugate, a portion of the immunoconjugate which has a property rendering its presence detectable. For example, the immunoconjugate may be labeled with a radioactive isotope which permits cells in which the immunoconjugate is present to be detected in immunohistochemical assays.

The terms "effector moiety" or "effector molecule" mean the portion of an immunoconjugate intended to have an effect on a cell targeted by the targeting moiety or to identify the presence of the immunoconjugate. Thus, the effector moiety can be, for example, a therapeutic moiety, a toxin, a radiolabel, or a fluorescent label.

"Immunotoxins" are chimeric proteins in which, typically, a "toxin" is coupled, directly or through a linker, to a "targeting moiety" to generate potent cell-type-specific-killing reagents. In the context of the present invention, the term "targeting moiety" refers to a portion of an immunotoxin that is able to target the toxin or toxic moiety to a cell or tissue of interest, usually by specifically binding to its corresponding target (*e.g*., a receptor on a cell surface). Examples of "targeting moieties" include, but are not limited to, receptor binding ligands, and antibodies or antibody fragments which retain antigen recognition capability, such as a scFv, a dsFv, an Fab, or an F(ab')₂. Thus, for example, where the targeting moiety is an antibody, the immunotoxin will specifically bind to cells bearing the epitope to which the antibody is directed. As used herein, the terms "targeting moiety" and "targeting molecule" are used interchangeably.

"Toxins" are typically cytotoxic enzymes, usually from plants and bacteria, and include abrin, ricin, *Pseudomonas* exotoxin A (PE), Diphtheria toxin (DT), botulinum toxin, and modified and mutated forms of these toxins which retain cytotoxic properties when targeted to cells of interest. For example, PE and DT are highly toxic compounds that typically bring about death through liver toxicity. PE and DT, however, can be modified into a form for use in immunotoxins by removing the native targeting component of the toxin (e.g., domain Ia of PE or the B chain of DT) and replacing it with a different targeting moiety, such as an antibody. A "toxic moiety" is that portion of an immunotoxin responsible for the cytotoxicity of the overall molecule.

Immunotoxins act as a potent cell-killing agents by specifically targeting the toxin to cells bearing a particular target molecule recognized by the targeting moiety. There is an extensive literature on immunotoxins, including, *e.g*., Pastan *et al*., (1992) *Ann. Rev. Biochem. 61*: 331-354; Youle *et al*., (1980) *Proc. Natl Acad. Sci. USA 77*:5483; Gilliland *et al*., (1980) *Proc. Natl Acad. Sci. USA 77*:4539; Krolick *et al*., (1980) *Proc. Natl Acad. Sci. USA 77*:5419; Griffin *et al*., (1988) *"Immunotoxins"* p 433, Boston/Dordrecht/Lancaster, Kluwer Academic Publishers; Vitetta *et al*., (1987) *Science 238:*1098; and Fitzgerald *et al*., (1989) *J. Natl. Cancer Inst. 81*:1455.

The targeting moiety and the toxin portions of immunotoxins of the invention are attached by a molecule termed a "connector" or "linker". As used herein, the terms "connector" and "linker" are interchangeable. Typically, a connector has no specific biological activity other than to join the proteins or to preserve some minimum distance or other spatial relationship between them. However, the constituent amino acids of a connector may be selected to influence some property of the molecule such as the folding, net charge, or hydrophobicity of the molecule. The linker is capable of forming covalent bonds to both the antibody and to the toxin molecule.

The terms "recombinant DNA," "recombinant nucleic acid" or "recombinantly produced DNA" refer to DNA which has been isolated from its native or endogenous source and modified either chemically or enzymatically by adding, deleting or altering naturally-occurring flanking or internal nucleotides. Flanking nucleotides are those nucleotides which are either upstream or downstream from the described sequence or sub-sequence of nucleotides, while internal nucleotides are those nucleotides which occur within the described sequence or subsequence.

The term "recombinant means" refers to techniques where proteins are isolated, the cDNA sequence encoding the protein identified and inserted into an expression vector. The vector is then introduced into a cell and the cell expresses the protein. Recombinant means also encompasses the ligation of coding or promoter DNA from different sources into one vector for expression of a chimeric protein, constitutive expression of a protein, or inducible expression of a protein.

The terms "recombinant protein," "recombinantly produced protein" or "recombinantly produced immunotoxin" refer to a peptide or protein produced using non-native cells that do not have an endogenous copy of DNA able to express the protein. The cells produce the protein because they have been genetically altered by the introduction of the appropriate nucleic acid sequence. The recombinant protein will not be found in association with proteins and other subcellular components normally associated with the cells producing the protein.

As used herein, "polypeptide", "peptide" and "protein" are used interchangeably and include reference to a polymer of amino acid residues. The terms apply to amino acid polymers in which one or more amino acid residue is an artificial chemical analogue of a corresponding naturally occurring amino acid, as well as to naturally occurring amino acid polymers. The terms also apply to polymers containing conservative amino acid substitutions such that the protein remains functional.

The term "residue" or "amino acid residue" or "amino acid" includes reference to an amino acid that is incorporated into a protein, polypeptide, or peptide (collectively "peptide"). The amino acid can be a naturally occurring amino acid and, unless otherwise limited, can encompass known analogs of natural amino acids that can function in a similar manner as naturally occurring amino acids.

The amino acids and analogs referred to herein are described by shorthand designations as follows in Table A:

**Table A:**

| Amino Acid Nomenclature | | |
|---|---|---|
| Name | 3-letter | 1-letter |
| Alanine | Ala | A |
| Arginine | Arg | R |
| Asparagine | Asn | N |
| Aspartic Acid | Asp | D |
| Cysteine | Cys | C |
| Glutamic Acid | Glu | E |
| Glutamine | Gln | Q |
| Glycine | Gly | G |
| Histidine | His | H |
| Homoserine | Hse | - |
| Isoleucine | Ile | I |
| Leucine | Leu | L |
| Lysine | Lys | K |
| Methionine | Met | M |
| Methionine sulfoxide | Met (O) | - |
| Methionine | | |
| Norleucine | Nle | - |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

A "conservative substitution", when describing a protein refers to a change in the amino acid composition of the protein that does not substantially alter the protein's activity. Thus, "conservatively modified variations" of a particular amino acid sequence refers to amino acid substitutions of those amino acids that are not critical for protein activity or substitution of amino acids with other amino acids having similar properties (e.g., acidic, basic, positively or negatively charged, polar or non-polar, *etc.*) such that the substitutions of even critical amino acids do not substantially alter activity. Conservative substitution tables providing functionally similar amino acids are well known in the art. The following six groups in Table B each contain amino acids that are conservative substitutions for one another:

**Table B**

| | |
|---|---|
| 1) | Alanine (A), Serine (S), Threonine (T); |
| 2) | Aspartic acid (D), Glutamic acid (E); |
| 3) | Asparagine (N), Glutamine (Q); |
| 4) | Arginine (R), Lysine (K); |
| 5) | Isoleucine (I), Leucine (L), Methionine (M), Valine (V); and |
| 6) | Phenylalanine (F), Tyrosine (Y), Tryptophan (W). |

*See also*, Creighton, *PROTEINS*, W.H. Freeman and Company, New York (1984).

The terms "substantially similar" in the context of a peptide indicates that a peptide comprises a sequence with at least 90%, preferably at least 95% sequence identity to the reference sequence over a comparison window of 10-20 amino acids. Percentage of sequence identity is determined by comparing two optimally aligned sequences over a comparison window, wherein the portion of the polynucleotide sequence in the comparison window may comprise additions or deletions (*i.e.*, gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.

The phrase "disulfide bond" or "cysteine-cysteine disulfide bond" refers to a covalent interaction between two cysteines in which the sulfur atoms of the cysteines are oxidized to form a disulfide bond. The average bond energy of a disulfide bond is about 60 kcal/mol compared to 1-2 kcal/mol for a hydrogen bond. In the context of this invention, the cysteines which form the disulfide bond are within the framework regions of the single chain antibody and serve to stabilize the conformation of the antibody.

The terms "conjugating," "joining," "bonding" or "linking" refer to making two polypeptides into one contiguous polypeptide molecule. In the context of the present invention, the terms include reference to joining an antibody moiety to a toxin molecule. The linkage can be either by chemical or recombinant means. "Chemical means" refers to a reaction between the antibody moiety and the toxin moiety such that there is a covalent bond formed between the two molecules to form one molecule. More particularly, in the context of the invention, "chemical means" refers to reactions by which a linker molecule, usually a short peptide, is covalently bound on one end to a targeting molecule and on the other end to a toxin molecule.

### III. LINKER MOLECULES

The immunotoxins of the invention comprise a targeting moiety and a toxin moiety connected by a linker. Various linkers are known in the art, and share the properties of being unreactive under physiological conditions and of not interfering with the activity of the targeting or the toxin moieties. Where the antibody and the toxin molecule are polypeptides, the linkers may be joined to the constituent amino acids through their side groups (*e.g*., through a disulfide linkage to cysteine). However, in preferred embodiments, the linkers will be joined to the alpha carbon amino and carboxyl groups of the terminal amino acids.

In preferred embodiments, the linker is a peptide, and is preferably from 1 to 150 residues in length, more preferably 3 to 100 residues in length, and even more preferably about 3 to 50 or fewer residues in length. In even more preferred embodiments, the linker is from about 4 to about 20 amino acid residues in length and in some particularly preferred embodiments is about 6 to about 17 residues in length and in others is about 6 to 10 residues in length. Larger molecular weight molecules tend to have decreased tumor penetration, and the linker portion should not be so long as to reduce by more than 50%, or more preferably, by more than 25%, the ability of the immunotoxin to penetrate a tumor, compared to an immunotoxin having the same targeting and toxin moieties but a linker of 10 amino acid residues or fewer. Assays for determining tumor penetration by immunotoxins are well known in the art, and can be performed, for example, by radiolabeling the immunotoxin, introducing the immunotoxin into an animal having a solid tumor (such as a nude mouse into which cells of a human solid tumor have been introduced) and then sectioning and autoradiographing the tumor to determine how far into the tumor the immunotoxin has penetrated.

In especially preferred embodiments, the linkers are peptides about 4 to about 20 amino acid residues in length. In general, any peptide of this length can be used so long as it does not interfere with the proper folding or activity of the targeting moiety or of the toxin moiety of the immunotoxin. The effect of the linker on the activity of these moieties can be readily determined by assaying the binding of the targeting moiety to its target and by assaying the cytotoxicity of the toxic moiety on cells targeted by the targeting moiety. A decrease in binding affinity of the targeting moiety by more than 25% or a decrease in cytotoxicity of the toxin moiety by more than 25%, or both, indicate that the particular linker peptide tested is not suitable. Assays for determining the binding capabilities of numerous antibodies and ligands are known in the art. For example, the binding affinity of a ligand employed as the targeting molecule of the immunotoxin may be assayed by measuring the ability of the targeting molecule to displace a native ligand from its target substrate. This may be accomplished by labeling the native ligand and then incubating cells bearing the target receptor with a fixed amount of the labeled ligand and various concentrations of the ligand-containing immunotoxin. The amount of bound native ligand can be determined by detecting the amount of label bound to the target cell. Unlabeled native ligand can be run as a control.

One of skill will recognize that selection of the target cell is determined by the particular ligand. The particular label is chosen to minimally interfere with the binding of the labeled native ligand. Suitable labels are well known to those of skill in the art and include, but are not limited to radioactive labels (e.g.,.¹²⁵I, ³²P), fluorescent labels (e.g., fluorescein or rhodamine), and enzymatic labels (e.g., horseradish peroxidase). Cytotoxicity can be determined by standard assays, such as those taught in the Examples, below. The peptide can be mutated by site specific mutagenesis to introduce a residue with an appropriate functional group for reacting to PEG into the linker at any desired point, or to substitute a residue with such a functional group for any of the amino acid residues previously present in the linker. The chemistry of functional groups for conjugating PEG to amino acid residues is well known and taught, for example, in such references as Hermanson, *Bioconjugate Techniques*, Academic Press San Diego, CA (1996), at Chapter 15 and in Zalipsky et al., "Succinimidyl Carbonates of Polyethylene Glycol," *in* Dunn and Ottenbrite, eds., *Polymeric Drugs and Drug Delivery Systems*, American Chemical Society, Washington, D.C. (1991).

Cysteines are particularly preferred for being introduced into or substituted into a linker since cysteines permit ready conjugation of a PEG derivative by thiol chemistry, such as that set forth in the Examples. If desired, two or more cysteines can be added to the linker. Thiol chemistry is well known and is taught in such references as Hermanson, *supra*, and Zalipsky, *supra*.

A preferred linker molecule to mutagenize to contain a cysteine is ASGGPE (SEQ ID NO:1). In preferred embodiments, the linker peptide is selected from the following peptides, which are set forth in standard single letter code: ASGCGPE (SEQ ID NO:2), ASGCCGPE (SEQ ID NO:3), ASCGSGCPE (SEQ ID NO:4), ASCGTTGCPE (SEQ ID NO:8), KASGKKYGCKKGPE (SEQ ID NO:5), and KGGGCAGGPE (SEQ ID NO:6), with the first three listed being particularly preferred and the first one listed being the most preferred. While the particular peptides stated are preferred, the cysteines can be placed at other positions in the linker. For example, the preferred peptide ASGGPE (SEQ ID NO:1) could have cysteines placed as follows: CASGGPE (SEQ ID NO:9), ACSGGPE (SEQ ID NO:10), ASCGGPE (SEQ ID NO:11), ASGGCPE (SEQ ID NO:12), ASGGPCE (SEQ ID NO:13), and ASGGPEC (SEQ ID NO:14). More than one cysteine can be introduced into the linker, so that more than one residue of the linker can be PEGylated. Constructs employing these linkers can be tested for activity by standard assays, such as those set forth in the Examples, to confirm that the PEGylation does not interfere with the desired activity of the immunoconjugate.

### IV. PEGYLATION OF THE LINKER MOLECULE

In the immunoconjugates of the invention, the linker molecule is PEGylated. In general, any residue of the linker can be PEGylated, although it is generally preferable that the PEGylation by of a residue other than the first and last residues of the linker (that is, the residues covalently bound directly to the targeting and to the effector moieties), as PEGylation of these residues is more likely to interfere with folding or activity of the targeting or effector moieties. If desired, however, these residues can be PEGylated and the resulting immunoconjugate tested for targeting and effector activity, for example by the assays of toxicity of immunotoxins in the Examples.

Various procedures for PEGylating molecules are known in the art. Typically, such procedures employ derivatized PEGs which permit coupling PEG to proteins under the reaction conditions desired. A number of such derivatized PEGs are known, such as: N-hydroxysuccinimide (NHS) esters of PEG carboxylic acids, monomethoxyPEG₂-NHS, succinimidyl ester of carboxymethylated PEG (SCM-PEG), benzotriazole carbonate derivatives of PEG, glycidyl ethers of PEG, PEG *p*-nitrophenyl carbonates (PEG-NPC, such as methoxy PEG-NPC), PEG aldehydes, PEG-orthopyridyldisulfide, and carbonyldimidazol-activated PEGs. One of the most convenient PEG derivatives for bioconjugation is PEG-thiol. PEG-maleimide is particularly preferred for use in conjugating PEG to peptide linkers in the immunotoxins of the invention. All of the listed PEG derivatives are commercially available at various molecular weights. *See, e.g*.,Catalog, Polyethylene Glycol and Derivatives, 2000 (Shearwater Polymers, Inc., Huntsville, AL). This catalog also contains a description of the chemistry of the conjugation of each derivative and references to the literature concerning protocols for such conjugations. If desired, many of the above derivatives are available in a monofunctional monomethoxyPEG (mPEG) form. PEGylation of molecules such as the peptide linkers of the immunoconjugates herein is further discussed in, e.g., Hermanson, *Bioconjugate Techniques*, Academic Press San Diego, CA (1996), at Chapter 15 and in Zalipsky et al., "Succinimidyl Carbonates of Polyethylene Glycol," *in* Dunn and Ottenbrite, eds., *Polymeric Drugs and Drug Delivery Systems*, American Chemical Society, Washington, D.C. (1991).

Conveniently, PEG can be attached to a chosen position in the linker by site-specific mutagenesis. For example, the linker can be engineered to have a cysteine residue at the position desired to facilitate attachment of PEG. An exemplary procedure for attaching PEG to a linker by thiol chemistry to a cysteine engineered into a linker is set forth in the Examples. Other conjugation chemistry can, however, be employed. For example, although PEG-maleimide was used in the Examples, PEG-vinylsulfone or PEG-orthopyridyl-disulfide are activated PEGs which can bind to cysteine residues, the first by a thioether bond and the latter by a disulfide bond.

While conjugation to cysteine residues is one convenient method by which the linkers can be PEGylated, other residues can also be used if desired. The reactivity of the side chains of amino acid residues is well known, see, e.g., Feeney et al., "Chemical Modification of Proteins: An Overview," *in* Feeney and Whitaker, eds. *Modification of Proteins*, American Chemical Society, Washington, D.C. (1982), at Table 1. For example, acetic anhydride can be used to react with NH₂ and SH groups, but not COOH, S-S, or -SCH₃ groups, while hydrogen peroxide can be used to react with -SH and -SCH₃ groups, but not NH₂. Reactions can be conducted under conditions appropriate for conjugation to a desired residue in the linker employing chemistries exploiting the well established reactivities.

Where the linker is made by synthetic chemistry rather than by recombinant means, additional chemistries can be exploited. For example, a lysine residue can be added to the linker by forming the peptide bond using the epsilon-amino group rather than the usual α-amino group. The free α-amino group of such a lysine deprotonates at a lower pH than that of the free epsilon-amino group of lysines in the immunotoxin which are bound by the α-amino group. By controlling the pH of the reaction conditions, the lysine with the free α-amino group can be reacted with PEG without also PEGylating other lysines in the linker or, if the PEGylation is performed after the linker is incorporated into the immunotoxin, with other lysines in the immunotoxin.

In general, the PEG added to the linker should range from a molecular weight (Mw) of several hundred Daltons to about 100 kD. Larger Mw PEG may be used, but may result in some loss of yield of PEGylated protein. The purity of larger PEG molecules should also be watched, as it may be difficult to obtain larger Mw PEG of purity as high as that obtainable for lower Mw PEG. It is preferable to use PEG of at least 85% purity, and more preferably of at least 90% purity, 95% purity, or higher. Larger Mw PEG may also display different characteristics in terms of target cell binding, translocation (where the effector molecule is to be internalized) and, in the case of a PE-based immunotoxin, in terms of the ADP-ribosylation by the toxin. These characteristics may vary for different types of tumors (hematologic tumors versus carcinomas); they may be readily tested by standard assays, such as those taught in the Examples.

Preferably, the Mw of the PEG should range from about 1 kD to about 30 kD and even more preferably from about 3 kD or more to about 30 kD. In particularly preferred embodiments, the Mw should range from at or about 5 kD to about 20 kD. Various derivatives of PEG are commercially available in these molecular weights. For example, monomethyl ether of PEG is available from Shearwater Polymers, Inc. in Mw of 1 kD, 2 kD, 3 kD, 5 kD, 10 kD, 12 kD and 20 kD, while various forms of PEG succinimidyl propionate are available in 2 kD, 3,400 D, 5 kD, and 20 kD Mws. As noted above, larger Mw PEGs can also be used, but PEGs up to 30 kD are preferred simply because they are more readily available commercially. As also noted, it is possible that PEGs of significantly higher Mw, for example, PEGs with Mw of over 100 kD, may be so large as to interfere to some degree with tumor penetration or other aspects of anti-tumor activity, pharmacokinetics, or other properties of the molecules. These concerns may be balanced, however, by a longer residence time of the PEGylated immunoconjugate in the target tissue. Any particular PEG can be tested by the assays set forth in the Examples and other assays known in the art to determine whether an immunoconjugate incorporating that PEG has a less desirable activity profile than that of the same immunoconjugate conjugated to a lower Mw PEG.

As should be clear from the description above, PEG is added to the linker as a substituent on a reactive side chain of an amino acid residue of the linker; PEG is not incorporated as an internal part of the linker polymer. PEG molecules can be conjugated to more than one amino acid residue of the linker, especially on linkers of 20 amino acids or more. It is generally desirable to have some distance along the length of the linker molecule between the residues to which the PEG is attached; a spacing of five residues is usually acceptable. It is usually not desirable to PEGylate more than four residues on a single linker.

Immunoconjugates with PEG conjugated to more than two residues should be tested (for example, by the assays set forth below) to confirm that they retain satisfactory activity. In general, if the immunoconjugate loses more than 25% of its activity compared to a similar immunoconjugate with only one linker residue conjugated to a PEG molecule (for example, if an immunotoxin loses more than 25% of its antitumor activity), than the number of residues of that immunoconjugate conjugated to PEG should be reduced.

### V. PRODUCTION OF IMMUNOCONJUGATES

Immunoconjugates include, but are not limited to, molecules in which there is a covalent linkage of a therapeutic agent to an antibody. A therapeutic agent is an agent with a particular biological activity directed against a particular target molecule or a cell bearing a target molecule. One of skill in the art will appreciate that therapeutic agents may include various drugs such as vinblastine, daunomycin and the like, cytotoxins such as native or modified *Pseudomonas* exotoxin or Diphtheria toxin, encapsulating agents, (*e.g*., liposomes) which themselves contain pharmacological compositions, radioactive agents such as ¹²⁵I, ³²P, ¹⁴C, ³H and ³⁵S and other labels, target moieties and ligands.

The choice of a particular therapeutic agent depends on the particular target molecule or cell and the biological effect is desired to evoke. Thus, for example, the therapeutic agent may be a cytotoxin which is used to bring about the death of a particular target cell. Conversely, where it is merely desired to invoke a non-lethal biological response, the therapeutic agent may be conjugated to a non-lethal pharmacological agent or a liposome containing a non-lethal pharmacological agent.

With the therapeutic agents and antibodies herein provided, one of skill can readily construct a variety of clones containing functionally equivalent nucleic acids, such as nucleic acids which differ in sequence but which encode the same effector molecule (EM) or antibody sequence. Thus, the present invention provides nucleic acids encoding antibodies and conjugates and fusion proteins thereof.

### A. Recombinant Methods

Nucleic acid sequences encoding immunoconjugates of the present invention can be prepared by any suitable method including, for example, cloning of appropriate sequences or by direct chemical synthesis by methods such as the phosphotriester method of Narang, *et al., Meth. Enzymol*. **68**:90-99 (1979); the phosphodiester method of Brown, *et al., Meth. Enzymol*. **68**:109-151 (1979); the diethylphosphoramidite method of Beaucage, *et al., Tetra. Lett*. **22**:1859-1862 (1981); the solid phase phosphoramidite triester method described by Beaucage & Caruthers, *Tetra. Letts*. **22**(20):1859-1862 (1981), *e.g*., using an automated synthesizer as described in, for example, Needham-VanDevanter, *et al. Nucl. Acids Res*. **12**:6159-6168 (1984); and, the solid support method of U.S. Patent No. 4,458,066. Chemical synthesis produces a single stranded oligonucleotide. This may be converted into double stranded DNA by hybridization with a complementary sequence, or by polymerization with a DNA polymerase using the single strand as a template. One of skill would recognize that while chemical synthesis of DNA is limited to sequences of about 100 bases, longer sequences may be obtained by the ligation of shorter sequences.

In a preferred embodiment, nucleic acid sequences encoding immunoconjugates are prepared by cloning techniques. Examples of appropriate cloning and sequencing techniques, and instructions sufficient to direct persons of skill through many cloning exercises are found in Sambrook, *et al*., MOLECULAR CLONING: A LABORATORY MANUAL (2ND ED.), Vols. 1-3, Cold Spring Harbor Laboratory (1989)), Berger and Kimmel (eds.), GUIDE TO MOLECULAR CLONING TECHNIQUES, Academic Press, Inc., San Diego CA (1987)), or *Ausubel, et al*. (eds.), CURRENT PROTOCOLS IN MOLECULAR BIOLOGY, Greene Publishing and Wiley-Interscience, NY (1987). Product information from manufacturers of biological reagents and experimental equipment also provide useful information. Such manufacturers include the SIGMA chemical company (Saint Louis, MO), R&D systems (Minneapolis, MN), Pharmacia LKB Biotechnology (Piscataway, NJ), CLONTECH Laboratories, Inc. (Palo Alto, CA), Chem Genes Corp., Aldrich Chemical Company (Milwaukee, WI), Glen Research, Inc., GIBCO BRL Life Technologies, Inc. (Gaithersberg, MD), Fluka Chemica-Biochemika Analytika (Fluka Chemie AG, Buchs, Switzerland), Invitrogen, San Diego, CA, and Applied Biosystems (Foster City, CA), as well as many other commercial sources known to one of skill.

Nucleic acids encoding native protein EM, linker peptides or antibodies can be modified to form EM, linker peptides, antibodies, or immunoconjugates in which the targeting moiety is linked by the linker to the EM. Modification by site-directed mutagenesis is well known in the art. Nucleic acids encoding protein EM or antibodies can be amplified by *in vitro* methods. Amplification methods include the polymerase chain reaction (PCR), the ligase chain reaction (LCR), the transcription-based amplification system (TAS), the self-sustained sequence replication system (3SR). A wide variety of cloning methods, host cells, and *in vitro* amplification methodologies are well known to persons of skill.

In a preferred embodiment, immunoconjugates are prepared by inserting the cDNA which encodes an anti-Tac scFv antibody into a vector which comprises the cDNA encoding the EM and cDNA encoding a linker. The insertion is made so that the scFv, the linker, and the EM are read in frame, that is in one continuous polypeptide which contains a functional Fv region, the linker, and a functional EM region.

Once the nucleic acids encoding an EM, antibody, or an immunoconjugate of the present invention are isolated and cloned, one may express the desired protein in a recombinantly engineered cell such as bacteria, plant, yeast, insect and mammalian cells. It is expected that those of skill in the art are knowledgeable in the numerous expression systems available for expression of proteins including *E. coli,* other bacterial hosts, yeast, and various higher eucaryotic cells such as the COS, CHO, HeLa and myeloma cell lines. In brief, the expression of natural or synthetic nucleic acids encoding the isolated proteins of the invention will typically be achieved by operably linking the DNA or cDNA to a promoter (which is either constitutive or inducible), followed by incorporation into an expression cassette. The cassettes can be suitable for replication and integration in either prokaryotes or eukaryotes. Typical expression cassettes contain transcription and translation terminators, initiation sequences, and promoters useful for regulation of the expression of the DNA encoding the protein. To obtain high level expression of a cloned gene, it is desirable to construct expression cassettes which contain, at the minimum, a strong promoter to direct transcription, a ribosome binding site for translational initiation, and a transcription/translation terminator. For *E. coli* this includes a promoter such as the T7, trp, lac, or lambda promoters, a ribosome binding site and preferably a transcription termination signal. For eukaryotic cells, the control sequences can include a promoter and preferably an enhancer derived from immunoglobulin genes, SV40, cytomegalovirus, and a polyadenylation sequence, and may include splice donor and acceptor sequences. The cassettes of the invention can be transferred into the chosen host cell by well-known methods such as calcium chloride transformation or electroporation for *E. coli* and calcium phosphate treatment, electroporation or lipofection for mammalian cells. Cells transformed by the cassettes can be selected by resistance to antibiotics conferred by genes contained in the cassettes, such as the *amp, gpt, neo* and *hyg* genes.

One of skill would recognize that modifications can be made to a nucleic acid encoding a polypeptide of the present invention (*i.e*., anti-Tac antibody, PE, or an immunoconjugate formed from their combination) without diminishing its biological activity. Some modifications may be made to facilitate the cloning, expression, or incorporation of the targeting molecule into a fusion protein. Such modifications are well known to those of skill in the art and include, for example, termination codons, a methionine added at the amino terminus to provide an initiation, site, additional amino acids placed on either terminus to create conveniently located restriction sites, or additional amino acids (such as poly His) to aid in purification steps.

In addition to recombinant methods, the immunoconjugates, EM, and antibodies or other ligands can also be constructed in whole or in part using standard peptide synthesis. Solid phase synthesis of the polypeptides of the present invention of less than about 50 amino acids in length may be accomplished by attaching the C-terminal amino acid of the sequence to an insoluble support followed by sequential addition of the remaining amino acids in the sequence. Techniques for solid phase synthesis are described by Barany & Merrifield, THE PEPTIDES: ANALYSIS, SYNTHESIS, BIOLOGY. VOL. 2: SPECIAL METHODS IN PEPTIDE SYNTHESIS, PART A. pp. 3-284; Merrifield, *et al. J. Am. Chem. Soc*. **85**:2149-2156 (1963), and Stewart, *et al*., SOLID PHASE PEPTIDE SYNTHESIS, 2ND ED., Pierce Chem. Co., Rockford, Ill. (1984). Proteins of greater length may be synthesized by condensation of the amino and carboxyl termini of shorter fragments. Methods of forming peptide bonds by activation of a carboxyl terminal end (*e.g.*, by the use of the coupling reagent N, N'-dicycylohexylcarbodiimide) are known to those of skill.

### B. Purification

Once expressed, the recombinant immunoconjugates, antibodies, effector molecules and linker molecules of the present invention can be purified according to standard procedures of the art, including ammonium sulfate precipitation, affinity columns, column chromatography, and the like *(see,* generally, R. Scopes, PROTEIN PURIFICATION, Springer-Verlag, N.Y. (1982)). Substantially pure compositions of at least about 90 to 95% homogeneity are preferred, and 98 to 99% or more homogeneity are most preferred for pharmaceutical uses. Once purified, partially or to homogeneity as desired, if to be used therapeutically, the polypeptides should be substantially free of endotoxin.

Methods for expression of single chain antibodies and/or refolding to an appropriate active form, including single chain antibodies, from bacteria such as *E. coli* have been described and are well-known and are applicable to the antibodies of this invention. See, *Buchner, et al., Anal. Biochem*. **205**:263-270 (1992); Pluckthun, *Biotechnology* **9**:545 (1991); Huse, *et al., Science* **246**:1275 (1989) and Ward, *et al*., *Nature* **341**:544 (1989), all incorporated by reference herein.

Often, functional heterologous proteins from *E. coli* or other bacteria are isolated from inclusion bodies and require solubilization using strong denaturants, and subsequent refolding. During the solubilization step, as is well-known in the art, a reducing agent must be present to separate disulfide bonds. An exemplary buffer with a reducing agent is: 0.1 M Tris pH 8, 6 M guanidine, 2 mM EDTA, 0.3 M DTE (dithioerythritol). Reoxidation of the disulfide bonds can occur in the presence of low molecular weight thiol reagents in reduced and oxidized form, as described in Saxena, *et al., Biochemistry* **9**: 5015-5021 (1970), incorporated by reference herein, and especially as described by Buchner, *et al., supra*.

Renaturation is typically accomplished by dilution (*e.g.*, 100-fold) of the denatured and reduced protein into refolding buffer. An exemplary buffer is 0.1 M Tris, pH 8.0, 0.5 M L-arginine, 8 mM oxidized glutathione (GSSG), and 2 mM EDTA.

As a modification to the two chain antibody purification protocol, the heavy and light chain regions are separately solubilized and reduced and then combined in the refolding solution. A preferred yield is obtained when these two proteins are mixed in a molar ratio such that a 5 fold molar excess of one protein over the other is not exceeded. It is desirable to add excess oxidized glutathione or other oxidizing low molecular weight compounds to the refolding solution after the redox-shuffling is completed.

### VI. IMMUNOTOXINS

In one aspect of the invention, the immunoconjugates are immunotoxins. Exemplary toxins include ricin, abrin, Diphtheria toxin and subunits thereof, saponin, gelonin, and ribosome inactivating protein, as well as botulinum toxins A through F. These toxins are readily available from commercial sources (*e.g.*, Sigma Chemical Company, St. Louis, MO). Diphtheria toxin is isolated from *Corynebacterium diphtheriae*. Ricin is the lectin RCA60 from *Ricinus communis* (Castor bean). The term also references toxic variants thereof, such as those taught in U.S. Patent Nos. 5,079,163 and 4,689,401. *Ricinus communis* agglutinin (RCA) occurs in two forms designated RCA₆₀ and RCA₁₂₀ according to their molecular weights of approximately 65 and 120 kD, respectively. (Nicholson & Blaustein, *J. Biochim. Biophys. Acta* 266:543 (1972)). The A chain is responsible for inactivating protein synthesis and killing cells. The B chain binds ricin to cell-surface galactose residues and facilitates transport of the A chain into the cytosol (Olsnes, *et al., Nature* 249:627-631 (1974) and U.S. Patent No. 3,060,165).

Abrin includes toxic lectins from *Abrus precatorius*. The toxic principles, abrin a, b, c, and d, have a molecular weight of from about 63 and 67 kD and are composed of two disulfide-linked polypeptide chains A and B. The A chain inhibits protein synthesis; the B-chain (abrin-b) binds to D-galactose residues (see, Funatsu, *et al., Agr. Biol. Chem*. 52:1095 (1988); and Olsnes, *Methods Enzymol*. 50:330-335 (1978)).

In preferred embodiments of the present invention, the toxin is *Pseudomonas* exotoxin (PE). The term "*Pseudomonas* exotoxin" as used herein refers to a full-length native (naturally occurring) PE or to a PE that has been modified. Such modifications may include, but are not limited to, elimination of domain Ia, various amino acid deletions in domains Ib, II and III, single amino acid substitutions and the addition of one or more sequences at the carboxyl terminus such as KDEL (SEQ ID NO:17) and REDL (SEQ ID NO:16). See e.g., Siegall, *et al., J. Biol. Chem*. 264:14256-14261 (1989). In a preferred embodiment, the cytotoxic fragment of PE retains at least 50%, preferably 75%, more preferably at least 90%, and most preferably 95% of the cytotoxicity of native PE. In particularly preferred embodiments, the cytotoxic fragment is more toxic than native PE.

Native *Pseudomonas* exotoxin A (PE) is an extremely active monomeric protein (molecular weight 66 kD), secreted by *Pseudomonas aeruginosa*, which inhibits protein synthesis in eukaryotic cells. The native PE sequence is provided in commonly assigned U.S. Patent No. 5,602,095, incorporated herein by reference. The method of action is inactivation of the ADP-ribosylation of elongation factor 2 (EF-2). The exotoxin contains three structural domains that act in concert to cause cytotoxicity. Domain Ia (amino acids 1-252) mediates cell binding. Domain II (amino acids 253-364) is responsible for translocation into the cytosol and domain III (amino acids 400-613) mediates ADP ribosylation of elongation factor 2. The function of domain Ib (amino acids 365-399) remains undefined, although a large part of it, amino acids 365-380, can be deleted without loss of cytotoxicity. *See* Siegall, *et al*., (1989), *supra*.

PE employed in the present invention include the native sequence, cytotoxic fragments of the native sequence, and conservatively modified variants of native PE and its cytotoxic fragments. Cytotoxic fragments of PE include those which are cytotoxic with or without subsequent proteolytic or other processing in the target cell (e.g., as a protein or pre-protein). Cytotoxic fragments of PE include PE40, PE38, and PE35.

In preferred embodiments, the PE has been modified to reduce or eliminate non-specific cell binding, frequently by deleting domain Ia. as taught in U.S. Patent 4,892,827, although this can also be achieved, for example, by mutating certain residues of domain Ia. U.S. Patent 5,512,658, for instance, discloses that a mutated PE in which Domain Ia is present but in which the basic residues of domain Ia at positions 57, 246, 247, and 249 are replaced with acidic residues (glutamic acid, or "E")) exhibits greatly diminished non-specific cytotoxicity. This mutant form of PE is sometimes referred to as PE4E.

PE40 is a truncated derivative of PE as previously described in the art. See, *Pai, et al*., *Proc. Nat'l Acad. Sci. USA* 88:3358-62 (1991); and Kondo, *et al., J. Biol. Chem*. 263:9470-9475 (1988). PE35 is a 35 kD carboxyl-terminal fragment of PE in which amino acid residues 1-279 have deleted and the molecule commences with a methionine at position 280 followed by amino acids 281-364 and 381-613 of native PE. PE35 and PE40 are disclosed, for example, in U.S. Patents 5,602,095 and 4,892,827.

In the most preferred embodiments, the cytotoxic fragment is PE38. PE38 is a truncated PE pro-protein composed of amino acids 253-364 and 381-613 which is activated to its cytotoxic form upon processing within a cell (see e.g., U.S. Patent No. 5,608,039, and Pastan et al., Biochim. Biophys. Acta 1333:C1-C6 (1997)).

As noted above, some or all of domain 1b may be deleted, and the remaining portions joined by a linker or directly by a peptide bond. Some of the amino portion of domain II may be deleted. And, the C-terminal end may contain the native sequence of residues 609-613 (REDLK) (SEQ ID NO:15), or may contain a variation found to maintain the ability of the construct to translocate into the cytosol, such as REDL (SEQ ID NO:16) or KDEL (SEQ ID NO:17), and repeats of these sequences. See, e.g., U.S. Patents 5,854,044; 5,821,238; and 5,602,095 and WO 99/51643. While in preferred embodiments, the PE is PE4E, PE40, or PE38, any form of PE in which non-specific cytotoxicity has been eliminated or reduced to levels in which significant toxicity to non-targeted cells does not occur can be used in the immunotoxins of the present invention so long as it remains capable of translocation and EF-2 ribosylation in a targeted cell.

### A. Conservatively Modified Variants of PE

Conservatively modified variants of PE or cytotoxic fragments thereof have at least 80% sequence similarity, preferably at least 85% sequence similarity, more preferably at least 90% sequence similarity, and most preferably at least 95% sequence similarity at the amino acid level, with the PE of interest, such as PE38.

The term "conservatively modified variants" applies to both amino acid and nucleic acid sequences. With respect to particular nucleic acid sequences, conservatively modified variants refer to those nucleic acid sequences which encode identical or essentially identical amino acid sequences, or if the nucleic acid does not encode an amino acid sequence, to essentially identical nucleic acid sequences. Because of the degeneracy of the genetic code, a large number of functionally identical nucleic acids encode any given polypeptide. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine) can be modified to yield a functionally identical molecule. Accordingly, each silent variation of a nucleic acid which encodes a polypeptide is implicit in each described sequence.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid.

### B. Assaying for Cytotoxicity of PE

*Pseudomonas* exotoxins employed in the invention can be assayed for the desired level of cytotoxicity by assays well known to those of skill in the art. Exemplary toxicity assays are well known in the art. Thus, cytotoxic fragments of PE and conservatively modified variants of such fragments can be readily assayed for cytotoxicity. A large number of candidate PE molecules can be assayed simultaneously for cytotoxicity by methods well known in the art. For example, subgroups of the candidate molecules can be assayed for cytotoxicity. Positively reacting subgroups of the candidate molecules can be continually subdivided and reassayed until the desired cytotoxic fragment(s) is identified. Such methods allow rapid screening of large numbers of cytotoxic fragments or conservative variants of PE.

### C. Conjugating Targeting Moieties and Toxins

The toxic moiety and the antibody may be conjugated by chemical or by recombinant means *(see*, Rybak *et al*., (1995) *Tumor Targeting 1*:141). In the immunotoxins of the invention, the targeting and toxin moieties are connected through a linker molecule, which is usually a short peptide. Chemical modifications include, for example, derivitization for the purpose of linking the moieties to the linker, by methods that are well known in the art of protein chemistry. Suitable means of linking the toxic moiety and the recognition moiety to the linker comprise a heterobifunctional coupling reagent which ultimately contributes to formation of an intermolecular disulfide bond between the moieties and the linker. Other types of coupling reagents that are useful in this capacity for the present invention are described, for example, in U.S. Patent No. 4,545,985. Alternatively, an intermolecular disulfide may conveniently be formed between cysteines in each moiety which occur naturally or which are inserted by genetic engineering. The means of linking moieties may also use thioether linkages between heterobifunctional crosslinking reagents or specific low pH cleavable crosslinkers or specific protease cleavable linkers or other cleavable or noncleavable chemical linkages. The means of linking moieties of the immunotoxins may also comprise a peptidyl bond formed between moieties which are separately synthesized by standard peptide synthesis chemistry or recombinant means.

Possible genetic engineering modifications of the different proteins or portions of the immunotoxins include combination of the relevant functional domains of each into a single chain multi-functional biosynthetic protein expressed from a single gene derived by recombinant DNA techniques (*see*, for example, PCT published application WO/88/09344). Furthermore, recombinant DNA techniques can be used to link the recombinant toxin and the antibody to the linker. Accordingly, the immunotoxin can comprise a fused protein beginning at one end with the toxin and ending with the antibody at the other.

Methods of producing recombinant fusion proteins are well known to those of skill in the art. Thus, for example, Chaudhary *et al*., (1989) *Nature 339*:394; Batra *et al*., (1990) *J*. *Biol. Chem. 265*:15198; Batra *et al*., (1989) *Proc. Natl. Acad. Sci. USA 86*:8545; and Chaudhary *et al*., (1990) *Proc. Natl. Acad. Sci. USA 87*:1066 describe the preparation of various single chain antibody-toxin fusion proteins.

In general, producing immunotoxin fusion proteins involves separately preparing the DNA encoding the antibody, antibody fragment (*e.g.*, the Fv light and heavy chains), or ligand, the DNA encoding the linker, and the DNA encoding the toxin to be used. The sequences are then combined in a plasmid or other vector to form a construct encoding the particular desired chimeric protein. A simpler approach involves inserting the DNA encoding the particular antibody fragment (*e.g.*, the Fv region) or ligand into a construct already encoding the desired toxin and the linker.

Thus, for example, DNA encoding anti-Tac antibody/toxin immunotoxins is most easily prepared by inserting the DNA encoding the antibody V_{H} and V_{L} chains (Fv region) into constructs already containing DNA encoding the desired toxin and linker, or vice versa. The DNA sequence encoding the Fv region is inserted into the construct using techniques well known to those of skill in the art.

Mammalian cells have been used to express and secrete hybrid molecules such as antibody-cytokines (Hoogenboom *et al*., (1991) *Biochem. Biophys. Acta 1096*:345; Hoogenboom *et al*., (1991) *Mol. Immunol. 28*:1027) and antibody-enzyme (Casadei *et al*., (1990) *Proc. Natl. Acad. Sci. USA 87*:2047; *Williams et al*., (1986) *Gene 43*:319). In part, immunogenicity of foreign proteins is due to incorrect glycosylation patterns present on recombinant proteins. Therefore, eukaryotic cell lines are preferred over prokaryotic cells as the expressed proteins are glycosylated. Human-derived cell lines are particularly preferred in that these cells incorporate a sialic acid as the terminal glycoside. Cell lines such as the hamster CHO and BHK, as well as the HEK-293 human fibroblast line have been used to express recombinant human proteins.

Other genetic engineering modifications of the protein moieties of the immunotoxins of this invention include deletions of functionally unnecessary domains to reduce the size of the protein or to modify other parameters which facilitate production or utility, such as sequence changes to affect the solubility (*e.g*., cysteine to serine) or glycosylation sites. One skilled in the art would appreciate that many additional well known chemical and genetic modifications of proteins may be advantageously applied to any protein.

Preferred immunotoxins of the present invention are chimeric proteins containing as the toxic moiety a *Pseudomonas* exotoxin (PE) modified to reduce or eliminate non-specific cell binding. In particularly preferred embodiment, the toxic moiety is a truncated form of PE with a Mw of 38 kD (PE38) which has a deletion in its cell-binding domain, linked through a short peptide to an Fv fragment of the anti-human Tac monoclonal antibody that binds the IL-2 receptor α subunit. The construction of this unique linkage of the fusion protein between the toxin and the antibody eliminates the heterogeneity of chemically linked antibody/toxin protein conjugates. This, it is believed, may contribute to the increased potency and decreased immunogenicity of the immunotoxin. In especially preferred embodiments, the Fv portion of the anti-Tac antibody is linked to PE38 by a peptide connector having the sequence ASGGPE.

The invention includes nucleic acid constructs that encode the novel proteins described here. A nucleic acid construct is one which, when incorporated into an appropriate vector, is capable of replicating in a host. The constructs may be linked to other sequences capable of affecting the expression of the construct, such as promoters and enhancers.

### D. Uses of the Immunotoxins

The immunotoxins of the present invention may be utilized for the selective inhibition or killing of cells to which the immunotoxins is targeted by the targeting moiety by contacting the cells with the immunotoxin. This method is based on the appropriate selection of an antibody or ligand that binds to cell surface markers found specifically or predominantly on the type of cell that is to be selectively killed. For example, the immunotoxins of this invention include those comprising an antibody that binds to the IL-2 receptor α subunit (CD25). In preferred embodiment, the immunotoxins of the invention are used to kill or to inhibit the growth of cells of CD25+ hematologic malignancies, including, e.g., hairy cell leukemia (HCL), cutaneous T-cell lymphoma, chronic lymphocytic leukemia, Hodgkin's disease and adult T-cell leukemia. The immunotoxins can be added, for example, to cell cultures to purge the culture of cells of CD25+ malignancies or to inhibit the growth of such cells in the culture. The immunotoxins can further be used *in vivo* to inhibit the growth of malignant cells in an organism.

### VII. IMMUNOCONJUGATES OTHER THAN IMMUNOTOXINS

In addition to immunotoxins, the present invention provides other immunoconjugates with greater stability and reduced immunogenicity than those previously available. Thus, an antibody may be attached by a linker to a therapeutic agent or a label that is to be delivered directly to cells bearing an antigen specifically recognized by the antibody. Therapeutic agents include such compounds as nucleic acids, proteins, peptides, amino acids or derivatives, glycoproteins, radioisotopes, lipids, carbohydrates, or recombinant viruses. Nucleic acid therapeutic and diagnostic moieties include antisense nucleic acids, derivatized oligonucleotides for covalent cross-linking with single or duplex DNA, and triplex forming oligonucleotides.

Alternatively, the molecule linked to an antibody or other targeting moiety may be an encapsulation system, such as a liposome or micelle that contains a therapeutic composition such as a drug, a nucleic acid (*e.g*. an antisense nucleic acid), or another therapeutic moiety that is preferably shielded from direct exposure to the circulatory system. Means of preparing liposomes attached to antibodies are well known to those of skill in the art. See, for example, U.S. Patent No. 4,957,735; and Connor, *et al., Pharm. Ther*. 28:341-365 (1985).

### A. Detectable Labels

Antibodies or other ligands may optionally be covalently or non-covalently linked to a detectable label. Detectable labels suitable for such use include any composition detectable by spectroscopic, photochemical, biochemical, immunochemical, electrical, optical or chemical means. Useful labels in the present invention include magnetic beads (*e.g*. DYNABEADS), fluorescent dyes (*e.g*., fluorescein isothiocyanate, Texas red, rhodamine, green fluorescent protein, and the like), radiolabels (*e.g*., ³H, ¹²⁵I, ³⁵S, ¹⁴C, or ³²P), enzymes (*e.g*., horse radish peroxidase, alkaline phosphatase and others commonly used in an ELISA), and colorimetric labels such as colloidal gold or colored glass or plastic (*e.g*. polystyrene, polypropylene, latex, *etc.)* beads. Metal chelates can be linked to antibodies or other targeting moieties by methods well known in the art. See, e.g., Robert, B. et al., *Cancer Res*., 56:47584765 (1996). For example, bispecific antibodies have been developed that can bind to tumors and to metal chelates (Stickney, D. et al., *Cancer Res*. 51(24):6650-5 (1991); Rouvier, E. et al., *Horm. Res*. 47(4-6):163-167 (1997)). These chelates can contain radioisotopes, such as ¹¹¹indium, for imaging or for cell killing. The techniques known in the art can be applied to the present invention by, for example, using as a linker molecule an antibody bispecific for the chelate and for the targeting moiety of the immunoconjugate. In this instance, the bispecfic antibody can be engineered for PEGylation, for example by engineering a cysteine exposed on the surface of the framework region of the antibody,

Means of detecting such labels are well known to those of skill in the art. Thus, for example, radiolabels may be detected using photographic film or scintillation counters, fluorescent markers may be detected using a photodetector to detect emitted illumination. Enzymatic labels are typically detected by providing the enzyme with a substrate and detecting the reaction product produced by the action of the enzyme on the substrate, and colorimetric labels are detected by simply visualizing the colored label.

### B. Conjugation to the Antibody or Other Targeting Molecule

In a non-recombinant embodiment of the invention, effector molecules, *e.g*., therapeutic, diagnostic, or detection moieties, are linked to the targeting molecule through a linker molecule using any number of means known to those of skill in the art. Both covalent and noncovalent attachment means may be used.

The procedure for attaching an effector molecule to a linker will vary according to the chemical structure of the EM. Polypeptides typically contain a variety of functional groups; *e.g*., carboxylic acid (COOH), free amine (-NH₂) or sulfhydryl (-SH) groups, which are available for reaction with a suitable functional group on a peptide.

Alternatively, the antibody is derivatized to expose or to attach additional reactive functional groups. The derivatization may involve attachment of any of a number of molecules such as those available from Pierce Chemical Company, Rockford Illinois to permit such conjugations to occur.

In some circumstances, it is desirable to free the effector molecule from the targeting molecule when the immunoconjugate has reached its target site. Therefore, in these circumstances, immunoconjugates will comprise linkages which are cleavable in the vicinity of the target site. Cleavage of the linker to release the effector molecule from the linker molecule may be prompted by enzymatic activity or conditions to which the immunoconjugate is subjected either inside the target cell or in the vicinity of the target site. When the target site is a tumor, a linker which is cleavable under conditions present at the tumor site (*e.g*. when exposed to tumor-associated enzymes or acidic pH) may be used.

In view of the large number of methods that have been reported for attaching a variety ofradiodiagnostic compounds, radiotherapeutic compounds, drugs, toxins, and other agents to antibodies one skilled in the art will be able to determine a suitable method for attaching a given agent to an antibody or other polypeptide.

### VIII. PHARMACEUTICAL COMPOSITIONS AND ADMINISTRATION

The present invention also relates to compositions comprising the immunoconjugates of the present invention in a pharmaceutically acceptable carrier. In therapeutic applications, compositions are administered to a patient suffering from a disease, in an amount sufficient to slow the progress of the disease and its complications or to partially or completely arrest the disease or its complications. An amount adequate to accomplish one or more of these goals is defined as a therapeutically effective dose. Amounts effective for such uses will depend on the severity of the disease and the general state of the patient's health.

The immunoconjugates of the present invention may be administered by various means appropriate for different purposes. For example, immunotoxins of the invention may be used for contacting tumors in various parts of the body, according to methods known in the art for other immunotoxins (*see,* for example, Rybak *et al*., (1990) *Human Cancer Immunology*, in "*Immunology and Allergy Clinics of America*," W. B. Saunders, and references cited therein). The immunotoxins may be administered systemically by injection, most preferably intravenously, but also intramuscularly, subcutaneously, intrathecally, intraperitoneally, into vascular spaces, or into joints, *e.g.*, intraarticular injection.

Accordingly, the present invention also relates to pharmaceutical compositions comprising an immunoconjugate of this invention and a pharmaceutically acceptable carrier, particularly such compositions which are suitable for the above means of administration. The dose will be dependent upon the properties of the immunoconjugate employed, *e.g*., its activity and biological half-life, the concentration of the immunoconjugate in the formulation, the site and rate of dosage, the clinical tolerance of the patient involved, and the like as is well within the skill of the physician.

Single or multiple administrations of the compositions may be administered depending on the dosage and frequency as required and tolerated by the patient. For example, with respect to the administration of immunotoxins, the composition should provide a sufficient quantity of the immunotoxins of the invention to cause the progress of the disease or its complications to be slowed or stopped.

The compositions for administration can comprise a solution of the immunoconjugate, such as an immunotoxin, in a pharmaceutically acceptable carrier, preferably an aqueous carrier. A variety of aqueous carriers can be used, e.g., buffered saline and the like. These solutions are sterile and generally free of undesirable matter. These compositions may be sterilized by conventional, well known sterilization techniques. The compositions may contain pharmaceutically acceptable auxiliary substances as required to approximate physiological conditions such as pH adjusting and buffering agents, toxicity adjusting agents and the like, such as sodium acetate, sodium chloride, potassium chloride, calcium chloride, sodium lactate and the like. The pH of the solution is preferably in the range of pH 5 to 9.5, and is preferably pH 6.5 to 7.5. Typically, the immunoconjugates or derivatives thereof are in a solution having a suitable pharmaceutically acceptable buffer such as phosphate, tris (hydroxymethyl) aminomethane-HCl or citrate and the like. Buffer concentrations should be in the range of 1 to 100 mM. The solution of the immunoconjugate may also contain a salt, such as sodium chloride or potassium chloride in a concentration of 50 to 150 mM. An effective amount of a stabilizing agent such as albumin, a globulin, a detergent, a gelatin, a protamine or a salt of protamine may also be included and may be added to a solution containing the immunoconjugate or to the composition from which the solution is prepared. Systemic administration of the immunoconjugate is typically made every two to three days or once a week. Alternatively, daily administration is useful. Usually administration is by either intramuscular injection or intravascular infusion.

For intravenous administration, typical pharmaceutical compositions are about 0.01 to 100 mg per patient per day. Dosages from 0.1 up to about 1000 mg per patient per day may be used, particularly when the drug is administered to a secluded site and not into the blood stream, such as into a tumor or an organ within which a tumor resides. Actual methods for preparing parenterally administrable compositions will be known or apparent to those skilled in the art and are described in more detail in such publications as *Remingtons Pharmaceutical Science*, 15th Ed., Mack Publishing Co., Easton, PA, (1980).

Administration may also be intranasal or by other nonparenteral routes. The immunoconjugate may also be administered via microspheres, liposomes or other microparticulate delivery systems placed in certain tissues including blood.

The immunoconjugate may also be administered by aerosol to achieve localized delivery to the lungs. This is accomplished by preparing an aqueous aerosol, liposomal preparation or solid particles containing or derivatives thereof. A nonaqueous (e.g., fluorocarbon propellent) suspension could be used. Sonic nebulizers preferably are used in preparing aerosols. Sonic nebulizers minimize exposing the antibody or derivatives thereof to shear, which can result in degradation of the immunoconjugate.

Ordinarily, an aqueous aerosol is made by formulating an aqueous solution or suspension of the immunoconjugate together with conventional pharmaceutically acceptable carriers and stabilizers. The carriers and stabilizers will vary depending upon the requirements for the particular immunoconjugate, but typically include nonionic surfactants (TWEEN-20 OR -80®, PLURONIC-F128 OR -67®, or polyethylene glycol), innocuous proteins like serum albumin, or sorbitan esters, oleic acid, lecithin, amino acids such as glycine, buffers, salts, sugars or sugar alcohols. The formulations will be sterile. Aerosols generally will be prepared from isotonic solutions.

Further, the present invention relates to a method of selectively killing cells using a selective immunotoxin of the present invention having a ligand or an antibody specific for a target on the surface of the cells to be killed under conditions allowing binding of the ligand or antibody. Binding of the ligand or antibody to the surface marker on a cell causes the toxin (*e.g.*, PE or PE38) to selectively kill the cell. This method of the present invention may be used for cell separation *in vitro* by selectively killing unwanted types of cells, for example, in bone marrow prior to transplantation into a patient undergoing marrow ablation by radiation.

### IX. EXAMPLES

### A. Materials and Methods

### 1. Reagents

Methoxy-polyethylene glycol-maleimide (PEG-maleimide, Mw: 5,000 or 20,000) was obtained from Fluka or Shearwater Polymer. Other reagents and solvents were obtained from standard sources.

### 2. Bacterial strains and plasmids

*E. coli* DH5α (MAX efficiency) from Bethesda Research Laboratories (Gaithersburg, MD) was used for propagation of plasmids. *E. coli* CJ236 (Bio-Rad) was used for preparation of single-stranded uracil-containing phagemid DNA, to be used as template for oligodeoxynucleotide-directed mutagenesis. *E. coli* BL21 (λDE3), which carries the T7 RNA polymerase gene under the control of an inducible promoter on a λ prophage, was used as a host for expression of recombinant immunotoxins. Plasmid pRK79 encodes anti-Tac(Fv)-PE38 (LMB-2) (Kreitman *et al*. (1994), *supra*).

### 3. Mutagenesis of LMB-2

Mutagenesis of LMB-2 was done by Kunkel's method (Kunkel *et al*. (1991) *Methods Enzymol. 204*:125-139) with some modifications as previously described (Onda *et al*. (1999), *supra*). Mutations in the plasmid were confirmed by DNA sequencing.

### 4. Expression and purification of recombinant immunotoxins

The components of LMB-2 (native LMB-2) and cys1-LMB-2 were produced in *E. coli* BL21 (λDE3) containing the corresponding expression plasmids (pRK79 or mutant pRK79) as previously described *(Onda et al*. (1999), *supra*).

### 5. PEGylation of cys1-LMB-2 with one cysteine

A typical procedure for preparation of PEGylated LMB-2 is as follows. LMB-2 with a free thiol group in phosphate-buffered saline was allowed to react with 30-fold molar excess of 5-kDa or 20-kDa PEG-maleimide (PEG5K or PEG20K) at 25°C for 12 hrs. The reaction mixture was diluted with 20 volumes of Buffer A (20 mM Tris-HCl, 1 mM EDTA, pH 7.5), then directly loaded on a Q-sepharose column which was previously equilibrated with Buffer A. The column was washed with 50 volumes of Buffer A for removing uncoupled and uncoupled PEG5K or PEG20K, then eluted with Buffer B (1 M NaCl in Buffer A). The appropriate Q-sepharose (Amersham Pharmacia Biotech, Piscataway, NJ) fractions containing PEGylated LMB-2s were collected and diluted with 5 volumes of Buffer A, then loaded on a Mono-Q (Amersham Pharmacia Biotech, Piscataway, NJ) column which was previously equilibrated with Buffer A, to roughly separate PEGylated LMB-2s from unmodified cys1-LMB-2. The column was washed with 10 volumes of Buffer A, then eluted with a NaCl gradient (0-1 M) in Buffer A. After concentrating the appropriate Mono-Q fractions by Centricon YM-10 (AMICON, Beverly, MA), the crude PEGylated LMB-2s were separated on TSK G3000SW which was equilibrated and eluted with phosphate-buffered saline. The obtained PEGylated LMB-2s were extremely pure according to SDS-PAGE analysis.

The free sulfhydryl group content was determined by the DTNB method (Riddles *et al*. (1979) *Anal. Biochem. 94*:75-81).

The PEG content was assayed by a modification of the method of Childs (Childs (1975) *Microchem. J*. *20*:190-192). PEG5K or PEG20K was used as a standard.

### 6. Cytotoxicity assay

The specific cytotoxicity of native, cys1-LMB-2s and the PEGylated LMB-2s was assessed by protein synthesis inhibition assay on ATac-4 cells (Kreitman *et al*. (1994), *supra*).

### 7. Stability assay

The stability of LMB-2, cys1-LMB-2 and PEGylated LMB-2s was determined by incubating them at a final concentration of 10 µg/ml at 37°C in mouse serum. The amount of active immunotoxin remaining after different times of incubation was determined by ATac4 cytotoxicity assay.

### 8. Non-specific toxicity assay

Groups of four, five or seven female BALB/c mice (6-7 week old; about 20g) were injected intravenously with 200µl of increasing doses of LMB-2, cys1-LMB-2 and PEGylated LMB-2s. PBS containing 0.2% BSA was used as a diluent. The LD50 is the calculated dose of recombinant immunotoxin that kills 50% of the animals.

### 9. Antitumor study

Antitumor activities of the immunotoxins were determined in mice bearing ATac-4 tumors. ATac-4 cells (2 x 106) were inoculated sub-cutaneously on day 0 into nude mice (Athymic Ncr nu/nu; 7 week old; about 20g). Starting on day 4, mice were treated with intravenous injections of immunotoxins. PBS containing 0.2% BSA was used as a diluent, and 10 µg/mouse of PEG5K or PEG20K were used as controls. Therapy was given on days 4, 6, 8. Each treatment group consisted of five mice. Tumors were measured with a caliper every 2 days, and the volume of the tumor was calculated by using the formula: tumor volume (in mm3) = length x (width)2 x 0.4.

### 10. Pharmacokinetic assays

Normal female BALB/c mice (6-7 week old; about 20g) were injected intravenously with 2 µg of various immunotoxins. Blood samples were drawn at different times. The level of recombinant immunotoxin was measured in a bioassay in which diluted serum samples incubated with ATac-4 cells, and the ability of serum samples to inhibit protein synthesis was measured. The data was analyzed by an exponential curve fitting program RSTRIP (Version 5, MicroMath Scientific Software).

### 11. Immunogenicity assays

Female BALB/c mice (6-7 weeks old; about 20 g) in groups of five were immunized i.p. with LMB-2, cys1-LMB-2 and PEGylated LMB-2s at doses of 4 µg/mouse or 40 µg/mouse in PBS containing 0.2% mouse serum albumin (MSA). Two weeks after the first immunization, mice were once again immunized with the appropriate antigen. Blood samples were collected every 7 days after the first immunization. The specific IgG levels in serum were determined by enzyme-linked immunosorbent assay (ELISA), using goat anti-mouse IgG antibody conjugated to alkaline phosphatase (ALP) and p-nitrophenyl phosphate as substrate. The plates were coated with LMB-2.

### B. Results

### 1. Preparation and characterization of PEGylated LMB-2s

In LMB-2, the Fv portion of the anti-Tac antibody is linked to PE38 by a peptide connector (ASGGPE (SEQ ID NO:1)). To prevent loss of the antigen-binding, translocation and ADP-ribosylation functions of LMB-2 that are necessary for its specific cytotoxic activity against CD25+ tumor cells, a mutant form of LMB-2 with one cysteine in the peptide (ASGCGPE (SEQ ID NO:2)) that connects the Fv to PE38 was prepared (Figure 1A). The cysteine was used for site-specific PEGylation using free thiol chemistry. As shown in Figure 2, the PEGylated LMB-2 molecules ran as a single band on SDS PAGE when eluted from a TSK size exclusion column as a single peak. Figure 2 and Table 1 show that the specific *in vitro* cytotoxicity of cys1-LMB-2 against the ATac-4 cell (CD25+ cell line) was similar to that of native LMB-2. As expected each cys1-LMB-2 molecule contained one free thiol group whereas native LMB-2 did not. The yield of highly purified cys1-LMB-2 prepared from inclusion bodies was ∼7.0% which is the same as that of native LMB-2 (7.3%).

Purified cys1-LMB-2 with a free thiol group in the connector was site-specifically modified with PEG5K- or PEG20K-maleimide by the formation of a thioether bond (Figure 1B). After purification both types of PEGylated LMB-2s had similar cytotoxic activities and these were the same as the unmodified native and mutant LMB-2 (Figure 2 and Table 1). After site-specific PEGylation of cys1-LMB-2, the PEGylated LMB-2 did not have a free thiol group and instead contained about 1 mole of PEG/mole of protein.

### 2. Stability of PEGylated LMB-2 in mouse serum

The stabilities of various LMB-2 molecules were assessed by incubating them in mouse serum at 37°C for various periods of time (Figure 3). Both native and mutant LMB-2 were relatively stable for 1 hour, and then their cytotoxic activities diminished in a time dependent manner. In contrast, both PEGylated LMB-2s were stable. After 24 hrs, PEG5K- and PEG20K-LMB-2s retained about 40% and 50% of their initial activities.

### 3. Toxicity of PEGylated LMB-2s

For toxicity studies several different doses of each type of immunotoxin were injected intravenously into BALB/c mice. Almost all of the deaths occurred within 4 days after treatment. Table 2 indicates the number of dead mice and the total number of mice injected recorded 14 days after treatment. The LD50 of native and mutant LMB-2 was found to be about 0.5 mg/kg as shown in Table 1. By contrast, the PEGylated LMB-2s were well tolerated; the LD50s of PEG5K-LMB-2 and PEG20K-LMB-2 were about 3.0 mg/kg.

### 4. Antitumor activity of PEGylated LMB-2s

To assess antitumor activities ATac-4 cells were inoculated subcutaneously in nude mice on day 0. Treatment was started on day 4 when the tumors measured about 100 mm3. Animals were treated intravenously with three doses given on days 4, 6, and 8. The control groups received vehicle (PBS containing 0.2% BSA) or 10 µg of PEG5K or PEG20K. Native and mutant LMB-2s inhibited tumor growth in a dose dependent manner (Figure 4). The antitumor activities of both unPEGlyated LMB-2s were similar. The dose required to maintain the average tumor volume for 6 days at the level of the 1st injection was 0.025 mg/kg x 3 for LMB-2 and cys1-LMB-2 and 0.006 mg/kg x 3 for PEG5KLMB-2 and PEG20K-LMB-2. Complete regressions, which were defined as disappearance of tumor without regrowth after more than 50 days, were observed in 2 of 5 mice or 1 of 5 mice at the dose of 0.1 mg/kg x 3 of native or mutant LMB-2 respectively. At the 0.2 mg/kg x3 dose level one of five mice administered either native or mutant LMB-2 died from toxicity during the therapeutic period, but complete regressions were observed in all four remaining mice. The antitumor activities of both types of PEGylated LMB-2s were markedly improved. Complete regression was observed in 1 of 5 mice or 2 of 5 mice at the dose of 0.025 mg/kg x 3 of PEG5K- or PEG20K-LMB-2, respectively. At the dose of 0.05 mg/kg x 3, PEGylated LMB-2s caused complete regressions lasting over 50 days. When given by themselves PEG5K and PEG20K (10µg/mouse) had no antitumor activity. Overall, approximately 4 times the dose was required to produce comparable changes in tumor volume when either of the PEGylated LMB-2s is used.

### 5. Pharmacokinetics of PEGylated LMB-2s

BALB/c mice were injected intravenously with a single dose of 2 µg of each form of LMB-2. Blood was drawn at different times after the injection and assayed for immunotoxin levels on ATac-4 cells. As shown in Figure 5, the serum concentration profiles of native and mutant LMB-2s showed a biexponential elimination curve. The plasma half-lives of the unmodified LMB-2s were about 13 min (Table 3). In contrast, the serum concentration profiles of both PEGylated LMB-2s showed monoexponential elimination curves. The plasma half-live of PEG5K-LMB-2 was increased about 5-fold and that of PEG20K-LMB-2 about 8-fold. The area under the curve and mean residence time of both PEGylated LMB-2s was also substantially increased.

### 6. Immunogenicity of PEGylated in LMB-2s

To assess immunogenicity, BALB/c mice were immunized i.p. with each form of LMB-2 at a dose of 4 µg/mouse or 40 µg/mouse on day 0 and 14.

Serum samples were collect on day 21 after the first immunization, and mouse anti-LMB-2 IgG antibody levels in serum were determined by ELISA using native LMB-2 as the coating antigen. As shown in Figure 6, native LMB-2 and mutant LMB-2 at a dose of 4 µg/mouse x 2 markedly induced anti-LMB-2 IgG antibodies in mice. Higher doses of native LMB-2 and mutant LMB-2 could not be administered due to toxicity to the mice. In contrast, the immunogenicity of both PEGylated LMB-2s was found to be much lower. Native LMB-2 was used to detect the antibody response in this study, but very similar results were obtained when mutant LMB-2 or PEGylated LMB-2s were used to coat the plates.

### C. Discussion

Site-specific PEGylation of recombinant immunotoxin LMB-2 increases its stability, blood residence time and antitumor activity while decreasing its non-specific toxicity and immunogenicity. Overall the therapeutic window is increased by over 20-fold. These results have important clinical implications for the use of immunotoxins in patients.

In a recently completed clinical trial, LMB-2 was given to 35 patients and showed antitumor activity in a variety of malignancies. One complete response was observed in Hairy Cell Leukemia which has lasted over 22 months. Several different toxicities were observed in this trial. These included transaminase elevations due to liver damage, weight gain, hypotension, and fever. Toxic side-effects of recombinant immunotoxins are of two types. One type results from specific targeting of normal cells which express the same antigen as the tumor cells. To overcome this toxicity it may be necessary to discover a target antigen that is not expressed on normal cells. Identification of new tumor-related antigens is ongoing by analysis of the expressed sequence tag database analysis (Vasmatzis *et al*. (1998) *Proc. Natl. Acad. Sci. USA. 6*:300-304). The other type of toxicity arises from undefined non-specific adsorption and uptake by CD25-negative normal cells. In the Phase I trial with LMB-2 and in toxicity studies of LMB-2 using mice whose CD25 does not react with LMB-2 or monkeys whose CD25 does react, liver damage as evidenced by transaminase elevations in the blood was frequently encountered (Kreitman *et al*. (1999), *supra*; Kreitman and Pastan (1995), *supra*). If this type of toxicity can be reduced it should be possible to raise the dose of immunotoxin used to treat patients and obtain more frequent and substantial clinical responses. One approach to reducing non-specific toxicity is to restrict the distribution of immunotoxin from the blood to normal tissues by increasing its blood-residency time. Additionally, prolongation of plasma half-life may lead to enhanced antitumor efficacy (Onda *et al*. (1999), *supra*). PEGylation is a means to increase the blood-residency of LMB-2 and to enhance its therapeutic potency.

PEGylation of proteins increases their plasma half-lives by reducing proteolysis and restricting tissue-distribution such as glomerular filtration by the kidney and hepatic uptake (Pai *et al*. (1991) *Cancer Res.* 51:2808-2812; Chaffee *et al*. (1992) *J. Clin. Invest. 89*:1643-1651; Yabe *et al*. (1999) *J. Pharmacol. Exp. Ther. 289:1176-1184; Pyatak et al*. (1980) *Res. Commun. Chem. Pathol Pharmacol. 29*:113-127; Katre *et al*. (1987) *Proc. Natl. Acad. Sci. USA. 84*:1487-1491; Kitamura *et al*. (1990) *Biochem. Biophys. Res. Commun. 28*:1387-1394; Wang *et al*. (1993) *Cancer Res*. *53*:4588-4594; *Benhar et al*. (1994) *J. Biol. Chem. 269*:13398-133404; *Kuan et al*. (1994) *J. Biol. Chem. 269*:7610-7616). This effect is attributed to the increased molecular size and steric hindrance that result from attaching PEG to proteins. In most cases, the application of PEGylation has been limited to enzymes whose substrates are very small molecules, such as adenosine deaminase (ADA) and superoxide disumutase (SOD), because the attached PEG chain can sterically inhibit ligand-receptor and antigen-antibody binding which are based on macromolecular interactions (Benhar *et al*. (1994), *supra*; Kuan *et al*. (1994), *supra*). Additionally, PEGylation usually occurs at lysine residues, some of which may be in or near the active site of the protein, and this lysine modification by PEG is random and difficult to control (Tsutsumi *et al*. (1995) *Br. J. Cancer. 71*:963-968). Recombinant immunotoxins have three important functions (Pastan (1997), *supra*; Kreitman *et al*. (1994), *supra*; Kreitman *et al*. (1999), *supra*; Brinkmann *et al*. (1991), *supra*; Reiter *et al*. (1994), *supra*; Reiter *et al*. (1994), *supra*). These are antigen-binding, translocation and ADP-ribosylation of EF2. All three involve macromolecular interactions. Both the antigen-binding domain and the ADP-ribosylation domain contain lysine residues. To circumvent interference with macromolecular interactions a mutant LMB-2 with one cysteine residue in the non-functional connector between the Fv and PE moieties of LMB-2 was made and used the thiol of the cysteine for site-specific PEGylation (Figure 1).

The specific *in vitro* cytotoxicity of cys1-LMB-2 containing the extra cysteine residue against CD25+ tumor cells (ATac-4) is about the same as that of parental LMB-2 (Figure 2 and Table 1). cys1-LMB-2 was specifically PEGylated by formation of a thioether bond between the free thiol group in the connector region and a terminal maleimide group of PEG. The PEG5K- and PEG20K-LMB-2 produced retained full cytotoxic activity when compared with unmodified native and mutant LMB-2s (Figure 2 and Table 1). This is in part because LMB-2 is processed within the cell into two fragments by a proteolytic cleavage between amino acids 279 and 280 PE. The PEG remains with the Fv whereas most of the toxin (aa 280-613) is transported to the endoplasmic reticulum where it translocates to the cytosol and kills the cell. Usually, the activity of PEGylated proteins decreases with increasing molecular weight of the attached PEG because the steric hindrance due to the attached PEG increases with the increasing length of the PEG chain (Tsutsumi *et al*. (1996) *Br. J. Cancer 74*:1090-1095). In this case, however, PEG20K-LMB-2 had almost the same activity as PEG5K-LMB-2. This may be due to the relatively long distance between the PEG attachment site in the connector and the active sites of LMB-2. The stability of the PEGylated LMB-2s at 37°C in mouse serum which contains proteases and other inactivating activities was enhanced compared to the unmodified native and mutant LMB-2 (Figure 3). The PEG may sterically hinder LMB-2 from proteolytic attack or dissociation of the V_{H} and V_{L} chains which leads to aggregation and that PEG20K is more effective than PEG5K.

Both types of PEGylated LMB-2 showed a 3-4-fold higher antitumor activity than unmodified native and mutant LMB-2. In addition, their toxicity to mice was reduced about 6-fold (Figure 4, Tables 1 and 2). Consequently, PEGylation led to a 20-fold increase in therapeutic efficacy. PEGylation of LMB-2 effectively increased its blood-residency and AUC, which are due to an increase in molecular size and enhanced stability (Figure 5). The plasma half-lives were 5-fold longer with PEG5K-LMB-2 and 8-fold longer with PEG20K-LMB-2 than with unmodified LMB-2s. The increase in the antitumor activities is probably due to the increase in plasma half-life which allows more immunotoxin to enter the tumor over time through the leaky capillaries that are often found in tumors. It is well-known that the transport of PEGylated proteins from blood to normal tissues is limited and that the nonspecific cellular absorption and uptake of PEGylated molecules is reduced (Chaffee *et al*. (1992) *J. Clin. Invest. 89*:1643-1651; Illum *et al*. (1987) *Biomaterials 8*:113-117; Woodle and Lasic (1992) *Biochim. Biophys. Acta. 14*:171-199). Thus, the decrease in the toxicity of LMB-2 may be due, at least in part, to its reduced distribution from blood into normal tissues such as liver due to its increased molecular size (Chaffee *et al*. (1992), *supra*). Moreover, the non-specific binding and uptake of LMB-2 by normal cells may be suppressed by PEGylation which shields ionic interactions. PEGylated LMB-2s were also found to have decreased immunogenicity which could be due to reduced degradation by antigen-presenting cells such as macrophages, shielding of some epitopes of peptides after degradation (Wang *et al*. (1993) *Cancer Res. 53*:4588-4594) or the prevention of binding to receptors on B cells. Additionally, as mentioned above, the transport of PEGylated LMB-2s from blood to immune tissues such as spleen, bone marrow and lymphoid tissue may be limited and the non-specific binding and uptake of PEGylated proteins by antigen-presenting cells may be reduced. Detailed studies on tissue distribution and cell-binding will be helpful in clarifying these issues.

In summary, site-specific PEGylation of LMB-2 greatly improves its therapeutic potency as an antitumor agent in mice by increasing its plasma half-life and decreasing its nonspecific toxicity. The approach used with LMB-2 should be applicable to other recombinant immunotoxins and other immunoconjugates and increase their activity in patients.

**Table I**

| Characterization of PEGylated LMB-2 | | | | | |
|---|---|---|---|---|---|
| | Connector | Molar ratio of Free SH/LMB-2 | Molar ratio of PEG/LMB-2 | IC50 (pg/ml) | LD50 (mg/kg) |
| LMB-2 | ASGGPE (SEQ ID NO:1) | 0.1 | 0.0 | 50 | 0.51 |
| mutant | ASGCGPE | 1.1 | 0.0 | 60 | 0.54 |
| LMB-2 | (SEQ ID NO:2) | | | | |
| PEG_{5K}-mLMB-2 | | 0.0 | 1.1 | 60 | 2.92 |
| PEG_{20K}-mLMB-2 | | 0.0 | 1.0 | 70 | 3.09 |

**Table II**

| Acute in vivo toxicity of a single I.V. injection of PEGylated LMS-2s in mice Injected dose (mg protein/kg) | | | | | | | |
|---|---|---|---|---|---|---|---|
| Groups | 0.23 | 0.45 | 0.68 | 0.90 | 1.8 | 3.6 | 4.8 |
| LMB-2 | 1/7* | 3/7 | 5/7 | 7/7 | | | |
| mutant-LMB-2 | 0/7 | 3/7 | 5/7 | 7/7 | | | |
| PEG_{5K}-mLMB-2 | | 0/5 | | 0/7 | 2/7 | 5/7 | 4/4 |
| PEG_{20K}-mLMB-2 | | 0/5 | | 0/7 | 1/7 | 5/7 | 4/4 |

| | | | | | | | |
|---|---|---|---|---|---|---|---|
| *Value reported are number of mice dying divided by number of mice treated. | | | | | | | |

**Table III**

| Pharmacokinetic Parameters of Immunotoxins | | | |
|---|---|---|---|
| | T1/2 (min) | MRT (min) | AUC (ng min/ml) |
| LMB-2 | 13 | 34.1 | 31,604 |
| mutant LMB-2 | 13 | 35.3 | 31,385 |
| PEG_{5K}-LMB-2 | 66 | 67.9 | 111,589 |
| PEG_{20K}-LMB-2 | 106 | 75.3 | 145,835 |
| Values are calculated from data in Figure 5 | | | |

## Claims

1. A composition comprising a targeting molecule linked to an effector molecule through a connector molecule, and one or more polyethylene glycol (PEG) molecules conjugated to said connector molecule.

2. The composition of claim 1, wherein said targeting molecule is selected from a ligand, an antibody, and a fragment of an antibody, which fragment retains antigen recognition capability,

3. The composition of claim 2, wherein said antibody or fragment thereof specifically recognizes Tac.

4. The composition of claim 1, wherein said effector molecule is selected from the group consisting of a cytotoxin, a label, a radionuclide, a detectable label, a drug, a liposome, a nucleic acid, a recombinant virus, a glycoprotein, a ligand, and an antibody.

5. The composition of claim 4, wherein said cytotoxin is selected from the group consisting of *Pseudomonas* exotoxin (PE) or a fragment or mutant thereof which retains cytotoxic activity, *Diphtheria* toxin or a fragment or mutant thereof which retains cytotoxic activity, ricin, saponin, gelonin, ribosome inactivating protein, abrin, and botulinum A-F.

6. The composition of claim 5, wherein said PE is PE38.

7. The composition of claim 1, wherein said connector molecule is a peptide.

8. The composition of claim 7, wherein said connector molecule is selected from the group consisting of ASGCGPE (SEQ ID NO:2), ASGCCGPE (SEQ ID NO:3), ASCGSGCPE (SEQ ID NO:4), KASGKKYGCKKGPE (SEQ ID NO:5), ASCGTTGCPE (SEQ ID NO:8), and KGGGCAGGPE (SEQ ID NO:6).

9. The composition of claim 1, wherein said PEG molecule is substituted for a reactive group on an amino acid residue of said connector molecule.

10. The composition of claim 9, wherein said PEG molecule has a molecular weight of between 1 and 100 kD.

11. The composition of claim 9, wherein said PEG molecule has a molecular weight of between about 3 kD and about 50 kD.

12. The composition of claim 9, wherein said PEG molecule has a molecular weight of between about 5 kD and about 20 kD.

13. The composition of claim 1, wherein said targeting molecule is an anti-IL-2 receptor α subunit antibody known as anti-Tac, wherein said effector molecule is PE38, and said connector molecule is ASGCGPE (SEQ ID NO:2).

14. A composition comprising a composition of claim 1 in a pharmaceutically acceptable carrier.

15. A composition comprising a composition of claim 13 in a pharmaceutically acceptable carrier.

16. A method of increasing anti-tumor activity of an immunotoxin having a targeting moiety and a toxin moiety connected by a connector molecule, said method comprising covalently bonding a polyethylene glycol ("PEG") molecule to said connector molecule.

17. A method of claim 16 wherein two or more amino acid residues of said connector molecule are conjugated to PEG.

18. A method of claim 16, wherein said targeting molecule is selected from a ligand, an antibody, and a fragment of an antibody, which fragment retains antigen recognition capability.

19. The method of claim 18, wherein said antibody specifically recognizes Tac.

20. The method of claim 18, wherein said effector molecule is selected from the group consisting of a cytotoxin, a label, a radionuclide, a detectable label, a drug, a liposome, a nucleic acid, a recombinant virus, a glycoprotein, a ligand, and an antibody.

21. The method of claim 20, wherein said cytotoxin is selected from the group consisting of *Pseudomonas* exotoxin (PE) or a fragment or mutant thereof which retains cytotoxic activity, *Diphtheria* toxin or a fragment or mutant thereof which retains cytotoxic activity, ricin, saponin, gelonin, ribosome inactivating protein, abrin, and botulinum A-F.

22. The method of claim 21, wherein said PE is PE38.

23. The method of claim 18, wherein said connector molecule is a peptide.

24. The method of claim 23, wherein said connector molecule is selected from the group consisting of ASGCGPE (SEQ ID NO:2), ASGCCGPE (SEQ ID NO:3), ASCGSGCPE (SEQ ID NO:4), KASGKKYGCKKGPE (SEQ ID NO:5), ASCGTTGCPE (SEQ ID NO:8) and KGGGCAGGPE (SEQ ID NO:6).

25. The method of claim 18, wherein said PEG molecule is substituted for a reactive group on an amino acid residue of said connector molecule.

26. The method of claim 18, wherein said PEG molecule has a molecular weight of between 1 and 100 kD.

27. The method of claim 26, wherein said PEG molecule has a molecular weight of between about 3 kD and about 50 kD.

28. The method of claim 26, wherein said PEG molecule has a molecular weight of between about 5 kD and about 20 kD.

29. The method of claim 26, wherein said targeting molecule is an antibody which specifically recognizes an IL-2 receptor α subunit, said effector molecule is PE38, and said connector molecule is ASGCGPE (SEQ ID NO:2).

## Patentansprüche

1. Zusammensetzung, umfassend ein Targeting-Molekül, das durch ein Connectormolekül mit einem Effektormolekül verknüpft ist, und ein oder mehrere Polyethylenglycol (PEG) Moleküle, die mit dem Connectormolekül konjugiert sind.

2. Zusammensetzung nach Anspruch 1, wobei das Targeting-Molekül ausgewählt ist aus einem Liganden, einem Antikörper und einem Fragment eines Antikörpers, wobei das Fragment Antigen-Erkennungsvermögen behält.

3. Zusammensetzung nach Anspruch 2, wobei der Antikörper oder das Fragment davon Tac spezifisch erkennt.

4. Zusammensetzung nach Anspruch 1, wobei das Effektormolekül ausgewählt ist aus der Gruppe bestehend aus einem Cytotoxin, einem Marker, einem Radionuclid, einem nachweisbaren Marker, einem Arzneistoff, einem Liposom, einer Nucleinsäure, einem rekombinanten Virus, einem Glycoprotein, einem Liganden und einem Antikörper.

5. Zusammensetzung nach Anspruch 4, wobei das Cytotoxin ausgewählt ist aus der Gruppe bestehend aus *Pseudomonas* Exotoxin (PE) oder einem Fragment oder Mutanten davon, das cytotoxische Aktivität behält, *Diphtherie*toxin oder einem Fragment oder Mutanten davon, das cytotoxische Aktivität behält, Ricin, Saponin, Gelonin, Ribosom inaktivierendes Protein, Abrin und Botulinum A-F.

6. Zusammensetzung nach Anspruch 5, wobei das PE PE38 ist.

7. Zusammensetzung nach Anspruch 1, wobei das Connectormolekül ein Peptid ist.

8. Zusammensetzung nach Anspruch 7, wobei das Connectormolekül ausgewählt ist aus der Gruppe bestehend aus ASGCGPE (SEQ ID NR:2), ASGCCGPE (SEQ ID NR:3), ASCGSGCPE(SEQ ID NR:4), KASGKKYGCKKGPE (SEQ ID NR:5), ASCGTTGCPE (SEQ ID NR:8) und KGGGCAGGPE (SEQ ID NR:6).

9. Zusammensetzung nach Anspruch 1, wobei eine reaktive Gruppe auf einem Aminosäurerest des Connectormoleküls durch das PEG-Molekül substituiert wird.

10. Zusammensetzung nach Anspruch 9, wobei das PEG-Molekül ein Molekulargewicht von zwischen 1 und 100 kD aufweist.

11. Zusammensetzung nach Anspruch 9, wobei das PEG-Molekül ein Molekulargewicht von zwischen etwa 3 kD und etwa 50 kD aufweist.

12. Zusammensetzung nach Anspruch 9, wobei das PEG-Molekül ein Molekulargewicht von zwischen etwa 5 kD und etwa 20 kD aufweist.

13. Zusammensetzung nach Anspruch 1, wobei das Targeting-Molekül ein Anti-IL-2-Rezeptor-α-Untereinheit-Antikörper bekannt als Anti-Tac ist, wobei das Effektormolekül PE38 ist und das Connectormolekül ASGCGPE (SEQ ID NR:2) ist.

14. Zusammensetzung, die eine Zusammensetzung nach Anspruch 1 in einem pharmazeutisch verträglichen Träger umfasst.

15. Zusammensetzung, die eine Zusammensetzung nach Anspruch 13 in einem pharmazeutisch verträglichen Träger umfasst.

16. Verfahren zum Erhöhen der Anti-Tumor-Aktivität eines Immunotoxins, das eine Targetinggruppe und eine Toxingruppe aufweist, die durch ein Connectormolekül verbunden sind, wobei das Verfahren umfasst, dass ein Polyethylenglycol ("PEG") Molekül an das Connectormolekül kovalent gebunden wird.

17. Verfahren nach Anspruch 16, wobei zwei oder mehrere Aminosäurereste des Connectormoleküls mit PEG konjugiert sind.

18. Verfahren nach Anspruch 16, wobei das Targeting-Molekül ausgewählt ist aus einem Liganden, einem Antikörper und einem Fragment eines Antikörpers, wobei das Fragment Antigen-Erkennungsvermögen behält.

19. Verfahren nach Anspruch 18, wobei der Antikörper Tac spezifisch erkennt.

20. Verfahren nach Anspruch 18, wobei das Effektormolekül ausgewählt ist aus der Gruppe bestehend aus einem Cytotoxin, einem Marker, einem Radionuclid, einem nachweisbaren Marker, einem Arzneistoff, einem Liposom, einer Nucleinsäure, einem rekombinanten Virus, einem Glycoprotein, einem Liganden und einem Antikörper.

21. Verfahren nach Anspruch 20, wobei das Cytotoxin ausgewählt ist aus der Gruppe bestehend aus *Pseudomonas* Exotoxin (PE) oder einem Fragment oder Mutanten davon, das cytotoxische Aktivität behält, *Diphtherie*toxin oder einem Fragment oder Mutanten davon, das cytotoxische Aktivität behält, Ricin, Saponin, Gelonin, Ribosom inaktivierendes Protein, Abrin und Botulinum A-F.

22. Verfahren nach Anspruch 21, wobei das PE PE38 ist.

23. Verfahren nach Anspruch 18, wobei das Connectormolekül ein Peptid ist.

24. Verfahren nach Anspruch 23, wobei das Connectormolekül ausgewählt ist aus der Gruppe bestehend aus ASGCGPE (SEQ ID NR:2), ASGCCGPE (SEQ ID NR:3), ASCGSGCPE (SEQ ID NR:4), KASGKKYGCKKGPE (SEQ ID NR:5), ASCGTTGCPE (SEQ ID NR:8) und KGGGCAGGPE (SEQ ID NR:6).

25. Verfahren nach Anspruch 18, wobei eine reaktive Gruppe auf einem Aminosäurerest des Connectormoleküls durch das PEG-Molekül substituiert wird.

26. Verfahren nach Anspruch 18, wobei das PEG-Molekül ein Molekulargewicht von zwischen 1 und 100 kD aufweist.

27. Verfahren nach Anspruch 26, wobei das PEG-Molekül ein Molekulargewicht von zwischen etwa 3 kD und etwa 50 kD aufweist.

28. Verfahren nach Anspruch 26, wobei das PEG-Molekül ein Molekulargewicht von zwischen etwa 5 kD und etwa 20 kD aufweist.

29. Verfahren nach Anspruch 26, wobei das Targeting-Molekül ein Antikörper ist, der eine IL-2-Rezeptor-α-Untereinheit spezifisch erkennt, das Effektormolekül PE38 ist und das Connectormolekül ASGCGPE (SEQ ID NR:2) ist.

## Revendications

1. Composition comprenant une molécule de ciblage liée à une molécule effectrice par l'intermédiaire d'une molécule de connexion, et une ou plusieurs molécules de polyéthylèneglycol (PEG) conjuguées à ladite molécule de connexion.

2. Composition selon la revendication 1, dans laquelle ladite molécule de ciblage est choisie parmi un ligand, un anticorps, et un fragment d'un anticorps, ce fragment conservant une capacité de reconnaissance d'antigènes.

3. Composition selon la revendication 2, dans laquelle ledit anticorps ou fragment de celui-ci reconnaît spécifiquement le Tac.

4. Composition selon la revendication 1, dans laquelle ladite molécule effectrice est choisie dans le groupe constitué d'une cytotoxine, d'un marqueur, d'un radionucléide, d'un marqueur détectable, d'un médicament, d'un liposome, d'un acide nucléique, d'un virus recombiné, d'une glycoprotéine, d'un ligand, et d'un anticorps.

5. Composition selon la revendication 4, dans laquelle ladite cytotoxine est choisie dans le groupe constitué de l'exotoxine de *Pseudomonas* (PE) ou d'un fragment ou d'un mutant de celle-ci qui conserve l'activité cytotoxique, de la toxine diphtérique ou d'un fragment ou d'un mutant de celle-ci qui conserve l'activité cytotoxique, de la ricine, de la saponine, de la gélonine, d'une protéine inactivant le ribosome, de l'abrine, et des toxines botuliniques A à F.

6. Composition selon la revendication 5, dans laquelle ladite PE est la PE 38.

7. Composition selon la revendication 1, dans laquelle ladite molécule de connexion est un peptide.

8. Composition selon la revendication 7, dans laquelle ladite molécule de connexion est choisie dans le groupe constitué de la séquence ASGCGPE (SEQ ID NO: 2), de la séquence ASGCCGPE (SEQ ID NO: 3), de la séquence ASCGSGCPE (SEQ ID NO: 4), de la séquence KASGKKYGCKKGPE (SEQ ID NO: 5), de la séquence ASCGTTGCPE (SEQ ID NO: 8) et de la séquence KGGGCAGGPE (SEQ ID NO: 6).

9. Composition selon la revendication 1, dans laquelle ladite molécule de PEG est substituée à un groupe réactif sur un résidu d'acide aminé de ladite molécule de connexion.

10. Composition selon la revendication 9, dans laquelle ladite molécule de PEG a une masse moléculaire comprise entre 1 et 100 kD.

11. Composition selon la revendication 9, dans laquelle ladite molécule de PEG a une masse moléculaire comprise entre environ 3 kD et environ 50 kD.

12. Composition selon la revendication 9, dans laquelle ladite molécule de PEG a une masse moléculaire comprise entre environ 5 kD et environ 20 kD.

13. Composition selon la revendication 1, dans laquelle ladite molécule de ciblage est un anticorps dirigé contre la sous-unité α du récepteur IL-2, connu sous le nom d'anti-Tac, dans laquelle ladite molécule effectrice est la PE 38, et ladite molécule de connexion est la séquence ASGCGPE (SEQ ID NO: 2).

14. Composition comprenant une composition selon la revendication 1 dans un support acceptable sur le plan pharmaceutique.

15. Composition comprenant une composition selon la revendication 13 dans un support acceptable sur le plan pharmaceutique.

16. Procédé pour augmenter l'activité antitumorale d'une immunotoxine ayant un fragment de ciblage et un fragment de toxine liés par une molécule de connexion, ledit procédé comprenant la liaison covalente d'une molécule de polyéthylèneglycol (« PEG ») à ladite molécule de connexion.

17. Procédé selon la revendication 16, dans lequel deux résidus d'acide aminé ou plus de ladite molécule de connexion sont conjugués avec le PEG.

18. Procédé selon la revendication 16, dans lequel ladite molécule de ciblage est choisie parmi un ligand, un anticorps, et un fragment d'un anticorps, ce fragment conservant une capacité de reconnaissance d'antigènes.

19. Procédé selon la revendication 18, dans lequel ledit anticorps reconnaît spécifiquement le Tac.

20. Procédé selon la revendication 18, dans lequel ladite molécule effectrice est choisie dans le groupe constitué d'une cytotoxine, d'un marqueur, d'un radionucléide, d'un marqueur détectable, d'un médicament, d'un liposome, d'un acide nucléique, d'un virus recombiné, d'une glycoprotéine, d'un ligand, et d'un anticorps.

21. Procédé selon la revendication 20, dans lequel ladite cytotoxine est choisie dans le groupe constitué de l'exotocine de *Pseudomonas* (PE) ou d'un fragment ou d'un mutant de celle-ci qui conserve l'activité cytotoxique, de la toxine diphtérique ou d'un fragment ou d'un mutant de celle-ci qui conserve l'activité cytotoxique, de la ricine, de la saponine, de la gélonine, d'une protéine inactivant le ribosome, de l'abrine, et des toxine botuliniques A à F.

22. Procédé selon la revendication 21, dans lequel ladite PE est la PE 38.

23. Procédé selon la revendication 18, dans lequel ladite molécule de connexion est un peptide.

24. Procédé selon la revendication 23, dans lequel ladite molécule de connexion est choisie dans le groupe constitué de la séquence ASGCGPE (SEQ ID NO: 2), de la séquence ASGCCGPE (SEQ ID NO: 3), de la séquence ASCGSGCPE (SEQ ID NO: 4), de la séquence KASGKKYGCKKGPE (SEQ ID NO: 5), de la séquence ASCGTTGCPE (SEQ ID NO: 8) et de la séquence KGGGCAGGPE (SEQ ID NO: 6).

25. Procédé selon la revendication 18, dans lequel ladite molécule de PEG est substituée par un groupe réactif sur un résidu d'acide aminé de ladite molécule de connexion.

26. Procédé selon la revendication 18, dans lequel ladite molécule de PEG a une masse moléculaire comprise entre 1 et 100 kD.

27. Procédé selon la revendication 26, dans lequel ladite molécule de PEG a une masse moléculaire comprise entre environ 3 kD et environ 50 kD.

28. Procédé selon la revendication 26, dans lequel ladite molécule de PEG a une masse moléculaire comprise entre environ 5 kD et environ 20 kD.

29. Procédé selon la revendication 26, dans lequel ladite molécule de ciblage est un anticorps qui reconnaît spécifiquement une sous-unité α du récepteur IL-2, ladite molécule effectrice est la PE 38, et ladite molécule de connexion est la séquence ASGCGPE (SEQ ID NO: 2).
